# EUROPEAN PATENT APPLICATION

(11) **EP 0 588 036 A2**
(43) Date of publication of application: **23.03.1994**
(21) Application number: 93112165.1
(22) Date of filing: 29.07.1993
(51) Int. Cl.: C07K 17/10, C07K 3/18, C07K 15/00, A61K 37/02, C12P 21/08, C12N 15/12, C12N 5/16, C12N 15/85, G01N 33/548, C07K 17/12

(54) **Composition for isolating apamin receptors, apamin binding protein and uses thereof**

(30) Priority: 30.07.1992 US 922307; 30.07.1992 US 922604; 30.07.1992 US 923095
(71) Applicant: AMERICAN CYANAMID COMPANY, Wayne, NJ 07470-8426 (US)
(72) Inventor: Sokol, Patricia Tyson, Bedminster, New Jersey 07921 (US); Ziai, Mohammed Reza, Montvale, New Jersey 08543 (US); Chandra, Manik, River Vale, New Jersey 07675 (US)
(74) Representative: Wächtershäuser, Günter, Prof. Dr.

(57) **Abstract**

The present invention relates to a compositon of matter useful as an affinity matrix for isolating an apamin receptor from cellular material, comprising a derivatized agerose matrix having a free amino group covalently coupled to a free carboxyl group of apamin. It additionally relates to a protein, isolated from a vertebrate tissue sample, of approximately 80 KDa which specifically binds apamin, and to an approximately 55 KDa presumed degradation product of said protein, as well as antibodies which bind to said protein or to the presumed degradation product. The invention further relates to an isolated nucleic acid fragment, comprising a nucleic acid sequence encoding an apamin receptor protein, or a biologically active fragment thereof; and to host cells comprising said nucleic acid fragment.

## Description

Potassium (K) channels are integral membrane proteins of great molecular and functional diversity, present in practically all mammalian cells. These channels are primarily responsible for maintaining a resting membrane potential and are rapidly activated in response to an external depolarizing stimulus, binding of certain ligands, or changes in the intracellular concentration of calcium or ATP. In the excitable cells such as neurons or cardiac myocytes, K-channels determine the duration of the action potential, thus performing a vital function in the central nervous system and the cardiac functions (reviewed in Rudy, B., Neuroscience 25: 729-749, (1988); Halliwell, J. V., in Cook, N. S. (ed.), Potassium Channels: Structure, Classification, Function and Therapeutic Potential, Ellis Horwood Ltd., 348-372, (1990)). The calcium-activated K-channel sub-family consists of at least three discernible ionic currents: a large (BK), an intermediate (IK) and a small (SK) conductive channels (reviewed in Castle, M. A., et al., TINS, 12: 59-65, (1989); Haylett, B. G. and D. H. Jenkenson, in Cook, N. S. (ed.), Potassium Channels: Structure, Classification, Function and Therapeutic Potential, Ellis Horwood Ltd., 70-95, (1990); Latorre, R., et al., Ann. Rev. Physiol. 51: 385-399, (1989)). These K-channels are activated in response to a rise in the intracellular concentration of calcium [Ca²⁺]i. In addition to calcium [Ca²⁺]i, the BK and IK channels are also sensitive to the changes in the membrane potential, whereas SK-channel has no significant voltage sensitivity.

Functionally, the SK-channel is involved in the after hyperpolarization that follows action potentials in many neurons. These include the symphathetic ganglionic neurons, hippocampal neurons, neurosecretory neurons and spinal motor neurons, as well as the skeletal muscle cells (Rudy, B., Neuroscience, 25: 729-749, (1988); Latorre, R., et al., Annu. Rev. Physiol. 51:385-399, (1989); Pennefather, P. et al., Proc. Nat'l. Acad. Sci. USA 82: 3040-3044, (1985); Marty, A., TINS 12: 420-424 (1989); Lancaster, B., et al., Neurosci. 11: 23-30 (1991); and Strong, P. N., Pharmac. Ther. 46: 137-162, (1990)). Furthermore, the SK-channel has been suggested to play a major role in the spontaneous, transient outward currents in the tracheal smooth muscle cells (Saunders, H. H., et al., J. Pharmacol. Exp. Ther. 257: 1114-1119, (1991)), the inhibitory action of the α₁-adrenoceptors, neurotensin receptor and the P₂ of the ATP receptor (Haylett, B. G., et al., in Cook, N.S. (ed), Potassium Channels: Structure, Classification, Function and Therapeutic Potantial, 70-95, (1990) and Strong, P.N., Pharmac. Ther., 46: 137-162, (1990)).

The neuronal and the skeletal muscle SK-channel is specifically and avidly blocked by a bee venom-derived peptide toxin, apamin (Latorre, R., et al., Annu. Rev. Physiol. 51:385-399, (1989); Moczydlowski, E et al., J. Membrane Biol. 105: 95-111 (1988); Blatz, A.L., et al., J. Gen. Physiol. 84: 1-23 (1984); Blatz, A.L. et al., Nature 323: 718-720 (1986); and Blatz, A.L., et al., TINS 10: 463-467 (1987)). By all indications, the apamin receptor complex is either identical to, or closely associated with, the SK-channel. Apamin is an 18 amino acid neurotoxic peptide which has a single class of binding sites in the rat brain synaptosomes and rat brain slices with an apparent dissociation constant (K_{d}) of 10-25 pM (Habermann, E., et al., Eur. J Biochem. 94: 355-364 (1979); and Mourre, C., et al., Brain Res. 382: 239-249 (1986)). Apamin is also capable of a temperature dependent and high affinity (K_{d}=30-150 pM) binding to the detergent solubilized brain receptor sites (Seagar, J.J., et al., Biochemistry 25: 4051-4057 (1986); Seagar, M.J., et al., Neurosci. 7: 565-570 (1987); Schmid-Antomarchi, H., et al., Eur. J. Biochem. 142: 1-6 (1984); and Wu, K., et al., Brain Res. 360: 183-194 (1985)). The reported Bₘₐₓ value for the rat brain synaptosomes and brain slices is 10-30 fmol/mg protein (Mourre, C., et al., Brain Res. 382: 239-249 (1986); Seagar, J.J., et al., Biochemistry 25: 4051-4057 (1986); and Wu, K., et al., Brain Res. 360: 183-194 (1985)), while that for the detergent solubilized receptor ranges from 0.45 to 17 fmol/mg protein (Seagar, M.J. et al., Neurosci. 7: 565-570 (1987); and Schmid-Antomarchi, H., et al., Eur. J. Biochem. 142: 1-6 (1984)).

The polypeptide components of the apamin receptor have been studied by several groups. Crosslinking experiments using [¹²⁵I]apamin, followed by SDS-PAGE and autoradiography have indicated that the apamin binding proteins of the rat brain synaptosomal membrane consists of two protein species, a major 80-86 KDa protein and, in most reported preparations, a minor 50-59 KDa band (Seagar, J.J., et al., Biochemistry 25: 4051-4057 (1986); Seagar, M.J., et al., J. Biol. Chem. 260: 3895-3898 (1985); and Leveque, C., et al., FEBS Letters 275: 185-189 (1990)). Partial peptide mapping of the two protein bands using an anti-apamin anti-serum has shown that the smaller polypeptide is likely to be a proteolytic fragment of the larger protein and not an additional subunit of the apamin binding protein in the brain. Furthermore, in the plasma membrane of the cultured neurons or astrocytes, there are additional components with the ability to crosslink to [¹²⁵I]apamin. Crosslinking of [¹²⁵I]apamin to the membranes from the rat heart, liver and smooth muscle has also indicated that an 85-87 KDa polypeptide is the major labeled component of the apamin binding complex (Marqueze, B., et al., Biochem 169: 295-298 (1987)). A second 59 KDa protein was identified in the liver membrane only (Marqueze, B., et al., Biochem 169: 295-298 (1987)).

The blocking of the small conductance calcium activated potassium channel (sKca) results in prolongation of the action potential, while its activation by an increase in the intracellular calcium concentration accelerates the rate of hyperpolarization, thus shortening the duration of the action potential. In vascular smooth muscle cells (such as those in veins and arteries), activation of sKca results in the hyperpolarization of the smooth muscle membrane, which in turn results in the inhibition of the voltage-gated calcium channels. The inhibition of the latter may then lead to the relaxation of the blood vessels and lowering of the blood pressure. In the heart, modulation of sKca can be a potentially useful means to regulate an arrhythmic heart. In the nervous system, the hypocampus of Alzheimer's patients shows a drastic reduction in apamin density (Vaitukatis, J.L. et al., Methods in Enzymology 73: 46-52 (1981)). Further, apamin receptor in neurons has been reported to be involved in the process of learning and memory (Messier, C., et al., Brain Res. 551: 322-326 (1991)). Thus, manipulation of this receptor may also result in improving cognition. Notwithstanding the significant therapeutic potential manipulation of sKca may have, relatively little is known about the identity of the proteins involved in this channel. The present invention now provides key elements in the study of the potassium channel function.

### Summary of the Invention

The present invention relates to a composition comprising a derivatized agarose matrix with a free amino group covalently coupled to a free carboxyl group of apamin. This matrix is useful in isolation of apamin receptors from detergent solubilized cells or cell membranes. The invention also relates to a method for making the affinity matrix, comprising contacting the derivatized matrix with apamin in the presence of a carbodiimide under condensation reaction conditions, and preferably, in the presence of N-hydroxysulfosuccinimide. The invention also relates to a method for isolating an apamin receptor from cellular material; to a purified and isolated protein which specifically binds apamin; to a nucleic acid fragment comprising a sequence encoding an apamin receptor; as well as to the recombinantly produced apamin receptor per se. The purified and isolated protein is believed to represent the apamin receptor associated with the SK-channel. Such receptors are associated with calcium activated potassium channels in a variety of animal tissues, such as brain, skeletal, cardiac, vascular smooth muscle, pancreas, kidney and liver tissue. The invention additionally relates to host cells and recombinant vectors useful in expressing the apamin receptor gene and protein.

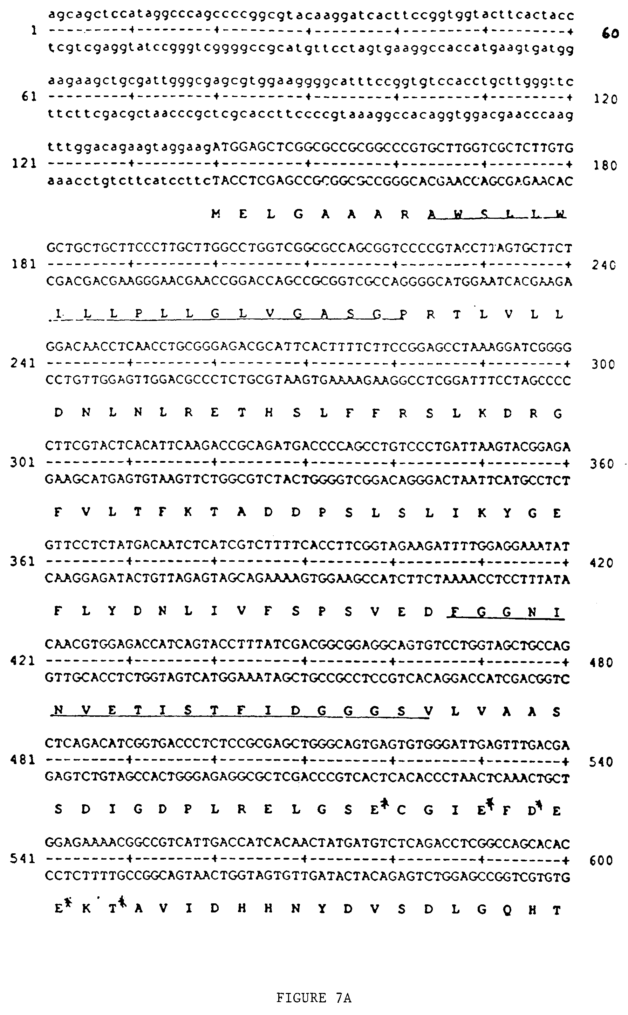

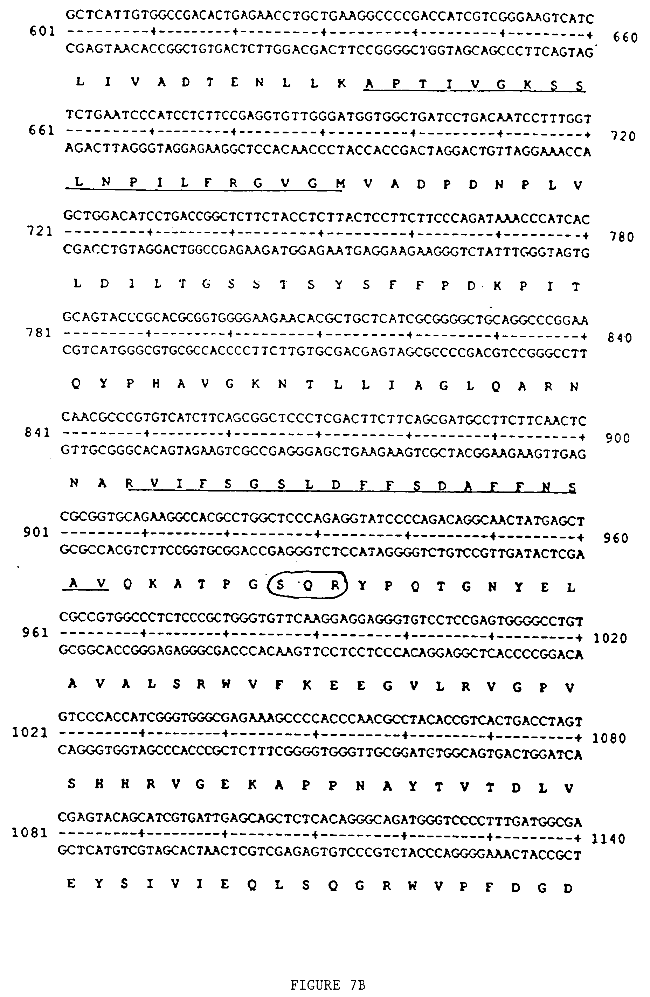

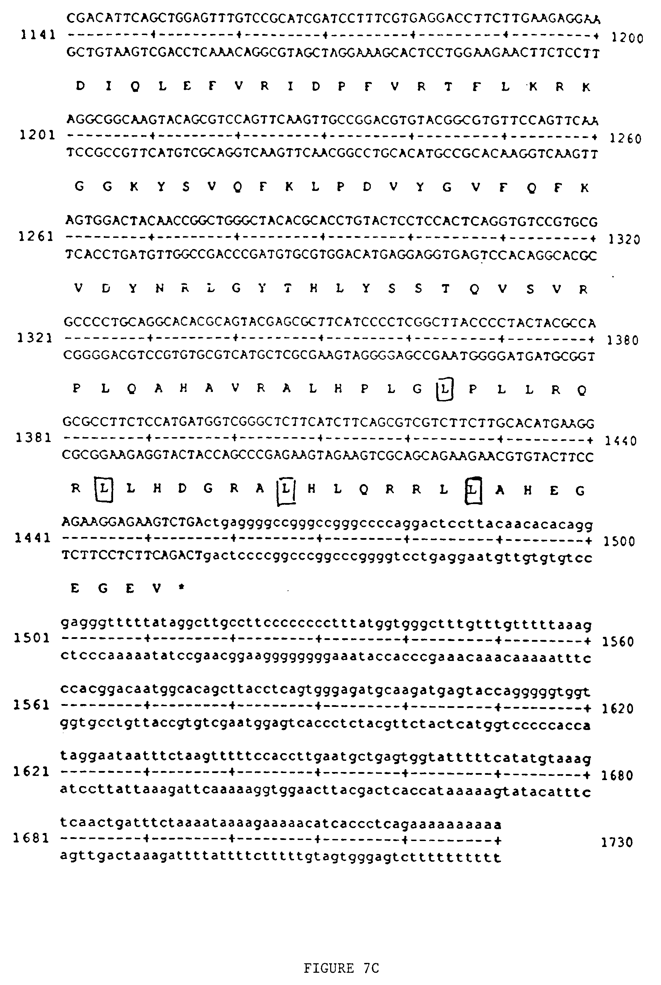

In the method of isolating the receptor of the current invention, the cellular material is detergent-solubilized; the detergent-solubilized material is contacted with the affinity matrix of the current invention at a maximum temperature of about 4°C, and for a time sufficient to permit binding of receptor to apamin; and the receptor is eluted from the matrix by raising the temperature to at least about 42°C. The protein of the current invention is approximately 80 KDa, and also yields an approximately 55 KDa presumed degradation product. The isolated protein, or immunogeneic or biologically active portions thereof, can be used to generate polyclonal antisera or monoclonal antibodies which are, in turn, useful in study of the structure and function of potassium channels, particularly the small conductance calcium activated potassium channel. By "biologically active" is meant proteins or fragments which are capable of eliciting production of antibodies capable of binding to the receptor, as well as proteins or fragments which are associated with the calcium activated potassium channels (such as BK or IK) but do not necessarily bind apamin. In another embodiment of the current invention, the sequence of the protein is now available; an exemplary sequence of Kcal 1.8, a porcine receptor, is provided in Figure 7A-7C (SEQ ID No.:2). The invention also encompasses any nucleotide sequence which hybridizes, under medium or high stringency conditions (as defined in the examples below), with a nucleotide sequence encoding the amino acid sequence of SEQ ID No.:2. The host cells of the current invention provide a convenient basis for development of screens designed to identify compounds which are capable of modulating activity of the receptor and thus, modulate the activity of the potassium channel. In the heart, modulation of this channel provides a means for regulating an arrythmic heart; thus, any drug that can open or close this potassium channel is considered a potential anti-arrythmic agent. Similarly, in vascular smooth muscle cells, such as those in veins and arteries, activation of the potassium channel results in hyperpolarization of the smooth muscle membrane, which in turn results in the inhibition of the voltage-gated calcium channels. The inhibition of the latter will then lead to relaxation of the blood vessels and lowering of blood pressure. The receptor is also associated with cognition functions. Receptor density decreases in Alzheimer's patients, and is involved in the process of learning and memory. Thus, compounds which activate the receptor may be useful in improving impaired cognitive function in Alzheimer's patients, or in enhancing memory and learning capacity. Therefore, a convenient system enabling the detection of compounds that modulate the potassium channel activity has the potential for identifying drugs with tremendous therapeutic utility. Also, the isolated nucleic acid sequence detectably labeled can be used as a diagnostic probe for Alzheimer's disease, by determining the level of expression of such receptors in perpheral neurons of individuals suspected of being affected.

### Brief Description of the Figures

Figure 1: SDS-PAGE and Coomassie stained analysis of the apamin binding protein, p80 isolated by affinity chromatography on a apamin-Sepharose 4B matrix. The affinity resin, loaded with the CHAPS solubilized bovine brain membranes and extensively washed, is sequentially eluted at 37°C (lane 1), 42°C (lane 2) and 55°C (lane 3). Lanes 4 and 5 show the profile of total CHAPS and SDS solubilized membrane proteins, respectively. The pre-stained molecular weight standards were phosphorylase B, bovine serum albumin, ovalbumin, carbonic anhydrase, soybean trypsin inhibitor and lysozyme.
Figure 2: Cross-linking of [¹²⁵I]apamin to purified p80. Purified p80 is incubated with [¹²⁵I]apamin, without (lane 1) or with (lane 2) an excess of unlabeled apamin. The complex is de-salted, cross-linked, separated by SDS-PAGE and autoradiographed.
Figure 3: Western immunoblotting of p80 in the rat brain membranes.
   (A) The antisera from mouse M1 (lane 1), mouse M2 (lane 2) and a non-immunized mouse (lane 3) are used in immunoblotting of a SDS-solubilized sample of the rat brain membrane proteins. The blots are developed as described below. Arrows indicate the position of two immunoreactive bands, p80 and p55.
   (B) The anti-p80 monoclonal antibody, D157, is used in immunoblotting of the SDS denatured sample of membranes from bovine brain (lane 1), rat brain (lane 2), rat heart (lane 3), rat kidney (lane 4) and rat liver (lane 5). Arrows indicate the position of p80 and p55.
   (C) The anti-p80 monoclonal antibody, D157, is used in immunoblotting of the SDS denatured sample of membrane from cultured human melanoma cells A375 (lane 1) and human medulloblastoma cells TE671 (lane 2). The position of p80 is indicated by an arrow.

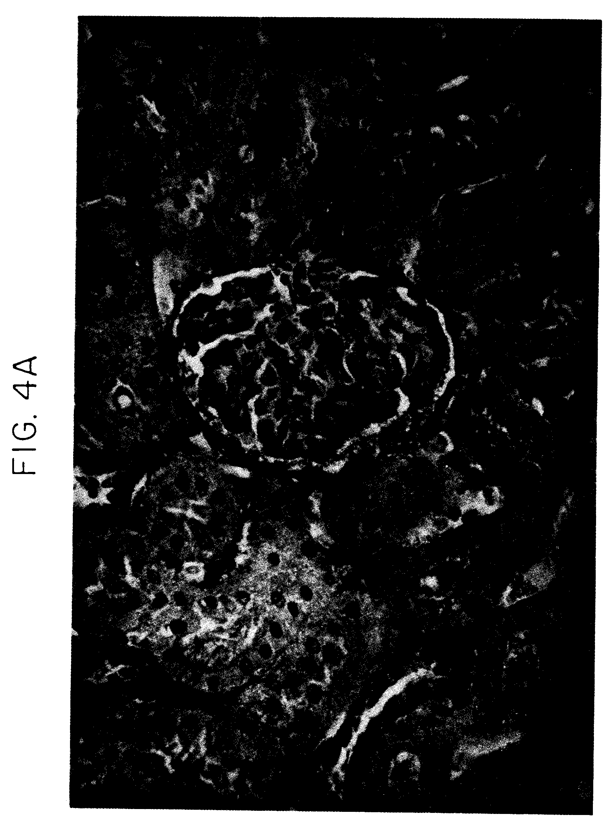

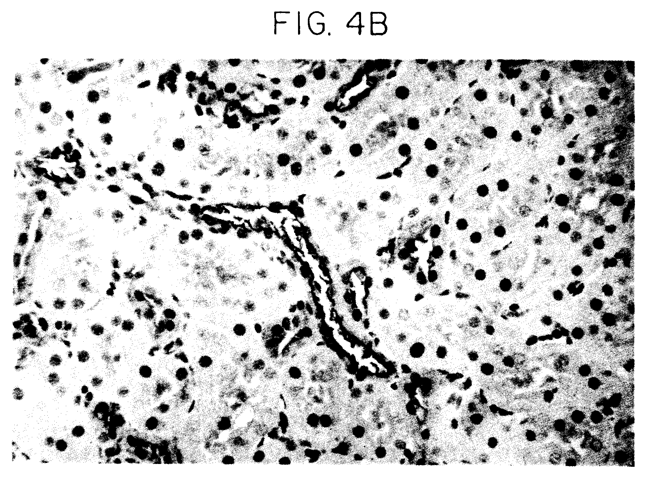

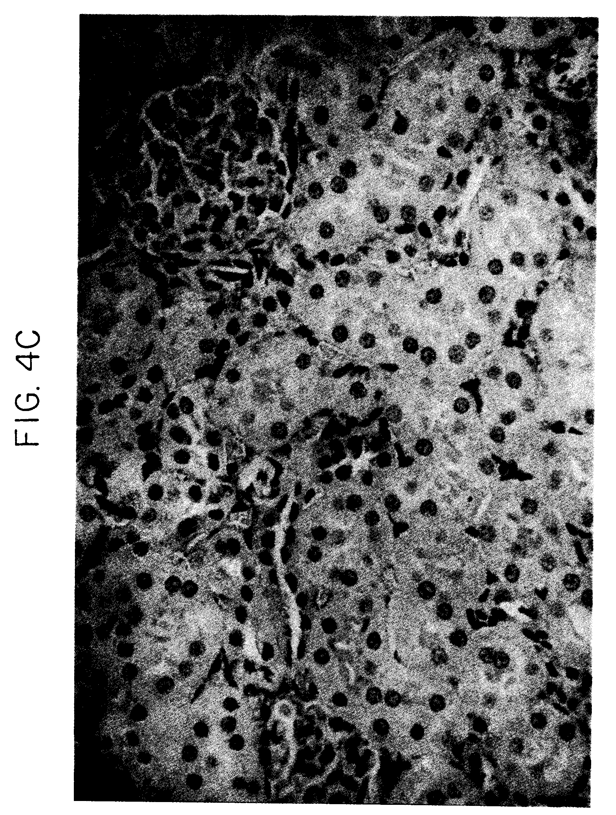

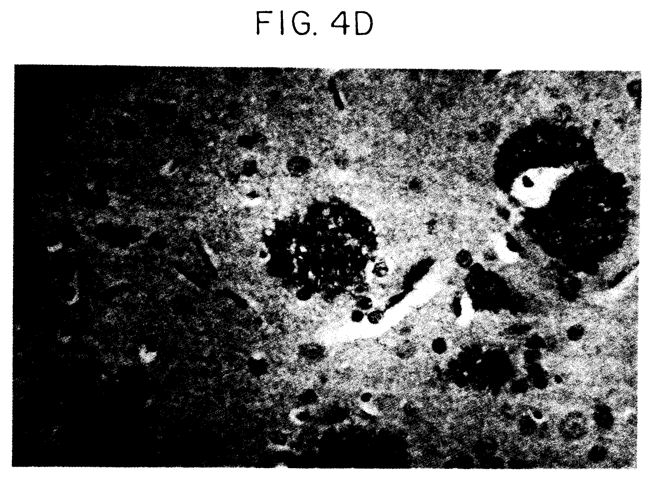

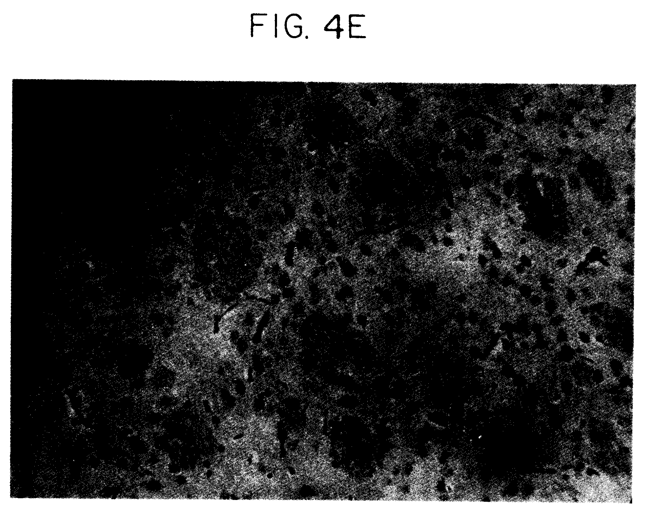

Figure 4: Immunocytochemical localization of p80 in rat tissues. The anti-p80 monoclonal antibody, D157, is used to stain a formalin-fixed, paraffin embedded section of the rat kidney (A & B). The control section is shown in panel C. Similarly, a section of the rat brain is either stained with monoclonal antibody D157 (D), or used as a control section (E).

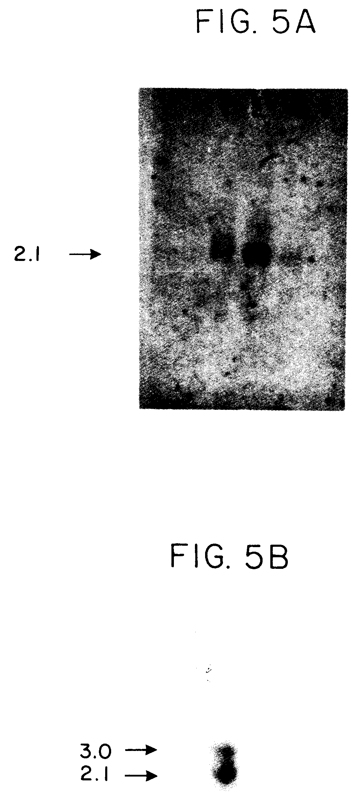

Figure 5: Northern blotting of the mRNA encoding the apamin receptor.
   (A) Poly A⁺-mRNA isolated from adult rat brain (lane 1), or bovine brain (lane 2) or porcine brain (lane 3) are separated on a denaturing agarose gel, blotted onto nitrocellulose, hybridized with ³²p-labelled Kcal 1.6 cDNA and autoradiographed.
   (B) Poly A⁺-mRNA isolated from the neonatal rat brain are separated on a denaturing agarose gel, blotted onto nitrocellulose, hybridized with ³²p-labelled Kcal 1.6 cDNA and autoradiographed. The arrows indicate the size (in kilobases) of the two hybridized mRNA bands.

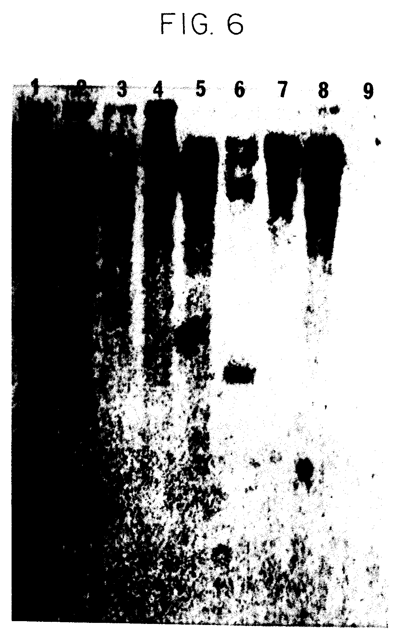

Figure 6: Genomic Southern hybridization analysis of Kcal 1.6. EcoRI cut-genomic DNA from human (lane 1), monkey (lane 2), rat (lane 3), mouse (lane 4), dog (lane 5), cow (lane 6), rabbit (lane 7), chicken (lane 8) and yeast (lane 9) are hybridized with ³²p-labelled Kcal 1.6 cDNA and autoradiographed.
Figure 7:
   (A), (B), (C): The nucleotide sequence and its amino acid translation of Kcal-1.8 cDNA. The underlined amino acids indicate the potential transmembrane domains of the protein. The oval represents the potential site for protein kinase C. The (*) indicate amino acids which form a potential calcium binding site.
   (D) The hydropathy plot for the protein encoded by Kcal-1.8 cDNA. The four putative but strong hydrophobic domains are indicated by arrows.

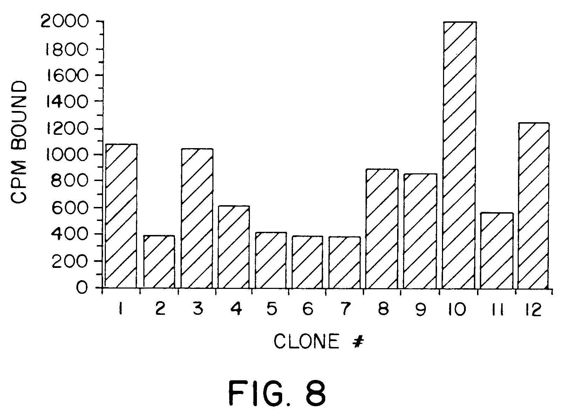

Figure 8: Binding of apamin to plasma membrane of CV-1 cells transfected with Kcal-1.8 cDNA in a pRC/CMV vector.

### Detailed Description of the Invention

The current invention pertains to an affinity matrix for isolating an apamin receptor; to a protein which specifically binds apamin, and a presumed degradation product; to antibodies which bind the protein or the presumed degradation product; to a nucleic acid fragment encoding the protein; and to host cells comprising said nucleic acid fragment, and progeny or derivatives thereof. Each of these embodiments are discussed sequentially below.

The efficient isolation of apamin binding proteins or receptors from cellular material requires the design of an affinity matrix adapted specifically for this purpose. The matrix necessary for this purpose should permit tight binding between the solid phase and detergent solubilized binding proteins, and should minimize the rate of dissociatlon between ligand and receptor.

Although the chromatographic matrix having a free amino group can comprise any solid support, including, for example, glass or polyacrylamide beads, the preferred support is a derivatized agarose matrix. In a preferred embodiment, the agarose bears a spacer arm with a free amino group. An example of a useful material for this purpose is EAH-Sepharose (Pharmacia), an agarose having a twelve carbon atom spacer arm with a free amino group. The spacer arm may be longer or shorter, but should not be so short as to potentially cause steric hindrance. The free carboxyl group of apamin is coupled to a terminal primary amino group of the deriviatized agarose matrix by carbodiimide condensation. In a preferred embodiment, N-hydroxysulfosuccinimide (sulfo-NHS) is added to the condensation reaction to increase the coupling of the ligand by stabilizing the reaction intermediates (Staros, J.V., et al., Analyt. Biochem. 156: 220-222 (1986)). Coupling is preferably conducted at a temperature or 4°, but may be conducted up to room temperature or higher, with a corresponding decrease in yield.

To make the receptors more "available" for binding to the affinity matrix, cells or cell membranes containing the receptor are solubilized in detergent. In order to determine which detergent would permit the binding of the apamin binding proteins to this affinity matrix, a number of ionic and non-ionic detergents are tested. The maximum degree of binding is observed when 1% w/v CHAPS or Lubrol-PX are used to solubilize cell membranes. A 1% v/v preparation of Triton X-100, Nonidet P-40, or SDS do not permit an efficient binding of the solid phase apamin to its binding proteins. In view of the dialysable nature of CHAPS, it is preferred as the solubilizing detergent.

In order to reduce the rate of the receptor-ligand dissociation, the incubation of the detergent-solubilized membranes with the apamin affinity matrix, as well as all subsequent washing steps, are performed at 4°C. The binding of apamin to its receptor has been shown to be stable at this temperature (Habermann, E. et al., Eur. J. Biochem. 94: 355-364 (1979)). Satisfactory binding occurs after an incubation of 4-16 hours.

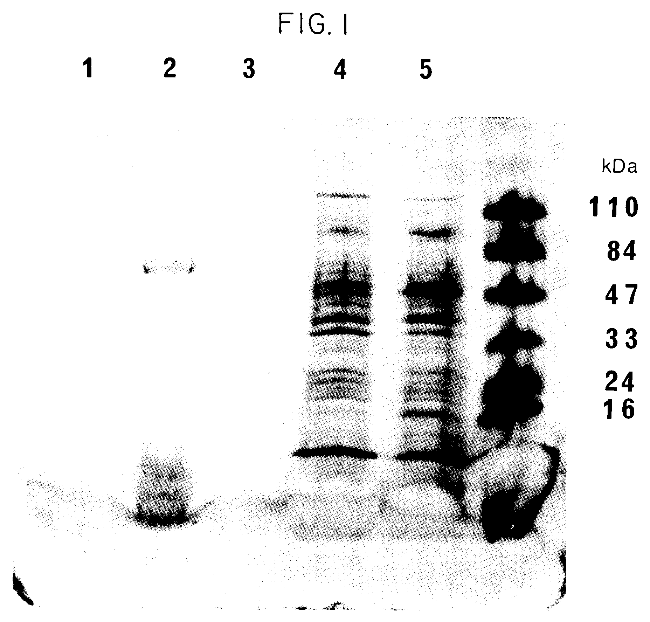

The elution of the apamin binding material from the affinity matrix, on the other hand, is carried out at 42°C-55°C, but at temperatures as high as 55°C there may be degradation of the receptor. The association of apamin with its receptor sites has been reported to be temperature dependent (Habermann, E. et al., Eur. J. Biochem. 94: 355-364 (1979)). As shown in Figure 1, incubation of the beads with the elution buffer for 15 minutes at 37°C does not lead to the elution of any protein (lane 1). A rapid shift of the temperature to 42°C for 15 minutes, however, results in the elution of a 80 KDa protein, p80 (lane 2). Further incubation of the affinity matrix at 55°C does not result in a detectable elution of any other proteins (lane 3). The yield of the isolated protein (p80) using this procedure is approximately 20 µg when 300 g of frozen bovine brain is processed.

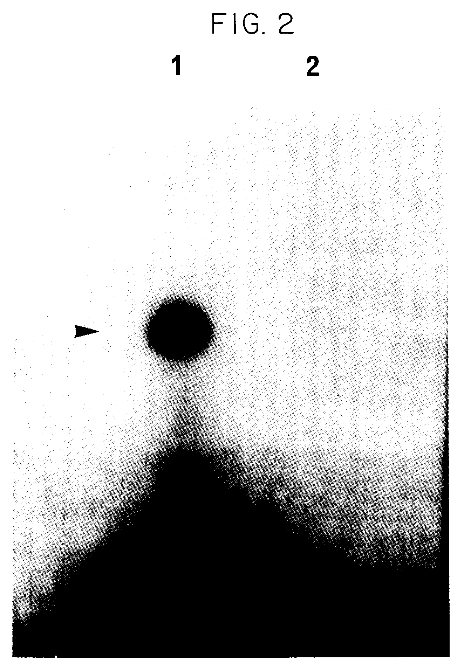

The specificity of the interaction between p80 and apamin is tested by a cross-linking experiment. To this end, the isolated p80 is incubated with [¹²⁵I]-apamin immediately following elution from the affinity matrix, with or without the inclusion of an unlabelled apamin. This is an essential precaution since the apamin binding ability of the isolated p80 is lost within 2 hours of the elution from the affinity matrix. This observation on the instability of the detergent-solubilized apamin binding sites is in complete agreement with the findings reported by Schmid-Antomarchi et al., (Eur. J. Biochem. 142: 1-6 (1984)). The [¹²⁵I]apamin-p80 complex is then separated from the unbound [¹²⁵I]apamin by gel filtration through a Sephadex G-50 column and the bound complex treated with the bifunctional cross-linker DMS. The cross-linked material shown in Figure 2 (lane 1), [¹²⁵I]apamin, is uniquely cross-linked to p80, while the excess and uncross-linked [¹²⁵I]apamin appears at the dye front. The interaction is considered specific, since the inclusion of the excess unlabelled apamin completely abrogates the cross-linking of [¹²⁵I]apamin to p80 (Figure 2, lane 2). In addition to DMS, the same experiment is repeated with four other chemically distinct bifunctional cross-linking reagents. Each time, the cross-linked protein band on the autoradiogram is superimposable to the p80 band in the coomassie stained gel.

Minor variations of the foregoing procedure which will produce substantially the same results will be apparent to those skilled in the art. This embodiment of the invention is further illustrated in examples 1-4.

The following discussion relates to an apamin-binding protein which is isolated from bovine brain. It will be recognized that similar proteins exist in other vertebrates, particularly other mammals, including humans. Additionally, such proteins also occur in other tissues, such as brain, heart, kidney, neuron, melanomas and neuroblastomas as shown below. Isolation of other binding protein subtypes, from alternate species and/or tissues can readily be achieved by the method described. Thus, the invention encompasses any protein with apamin binding specificity, regardless of source.

In a specific embodiment, an 80 kilodalton (KDa) apamin binding protein is isolated from bovine brain tissue by affinity purification. A crude membrane fraction from brain tissue is solubilized in detergent, and contacted with apamin Sepharose beads to isolate the apamin-binding protein. The affinity matrix is described above. SDS-PAGE separation of the eluate indicates the presence of an 80 KDa protein, hereinafter referred to as p80 (Figure 1, lane 2).

The specificity of the interaction between p80 and apamin is tested by a cross-linking experiment. The isolated p80 is incubated with radiolabelled apamin both in the presence and absence of an excess of unlabelled apamin. The labelled apamin-p80 complex is separated from unbound apamin, and treated with the bifunctional cross-linker dimethylsuberimidate (DMS). The cross-linked material, when analyzed by SDS-PAGE and autoradiographY, indicates a unique binding between the apamin and p80 (Figure 2, lane 2). Several repeats of the same experiment with other chemically distinct, bifunctional cross-linking reagents result in the production of a similar band.

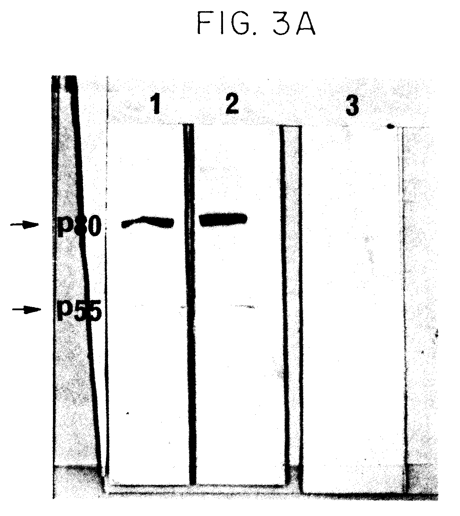

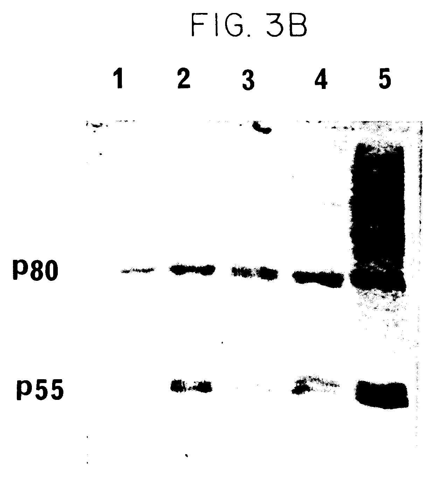

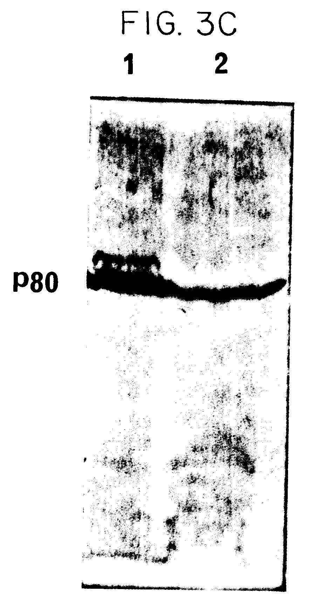

The protein thus purified is used to produce both polyclonal and monoclonal sera, as described in the following examples. The polyclonal antisera are used in Western blots of a rat brain synaptosomal preparation. The results indicate the antisera bind to two species, a major 80 KDa protein (p80), and a minor 55 KDa protein (p55). The proteins are not detectable with preimmune sera (Figure 3).

In order to determine whether the interaction with an 80 KDa and 55 KDa protein indicates that the serum is not monospecific, monoclonal antibodies are prepared and used to analyze the p80/p55 profile in a number of rat tissues. Results with two different monoclonal antibodies show the presence of both p80 and p55 in bovine brain, rat brain, rat heart, rat kidney and rat liver, with rat kidney and liver showing the p55 band as a doublet (Figure 3B). This indicates an immunological relationship between p80 and p55.

A deglycosylation experiment, together with a partial peptide mapping of the smaller apamin binding protein p55 and p80, has indicated that p55 is likely to be derived from p80 by proteolysis and not by deglycosylation (Leveque, C., et al., FEBS Letters 275: 185-189 (1990)). Further tests are conducted to determine whether p55 exists in the membrane of freshly isolated cultured cells. To this end, plasma membranes from human melanoma A375 and human medulloblastoma TE671 cells are prepared in the presence of a number of protease inhibitors, separated on SDS-PAGE and analyzed by immunoblotting using monoclonal antibody D157. As shown in Figure 3 (C), the antibody detects only one major 80 KDa band in the melanoma cells (lane 1) and TE671 cells (lane 2). A second band slightly larger than p80 is also observed in the melanoma cells (lane 1). In this experiment, no significant amount of p55 is detectable. This observation suggests that p55 is either a proteolytic fragment of p80 generated in vivo or the apamin binding protein complex(es) in the cultured cells is different from that found in the rat and bovine tissues. The latter possibility has been suggested by several groups (Seagar, M.J., et al., Biochemistry 25: 4051-4057 (1986); and Seagar, M.J., et al., J. Neurosci. 7: 565-570 (1987)). However, no direct evidence for this hypothesis has yet been produced and the existence of other sub-units of the apamin receptor complex remains a possibility. These results differ from those obtained by the cross-linking of [¹²⁵I]apamin to its binding proteins in one major respect. The results of the cross-linking experiments indicate that both p80 and p55 bind [¹²⁵I]apamin with an adequate affinity for the covalent bifunctional cross-linking agent to be effective ((Seagar, M.J., et al., Biochemistry 25: 4051-4057 (1986); Wu, K. et al., Brain Res. 360: 183-194 (1985); and Leveque, C., et al., FEBS Letters 275: 185-189 (1990)). The affinity chromatography using the apamin Sepharose 4B column suggests that only p80 binds to the solid phase apamin and no detectable levels of P55 can be found in the eluate (Figure 1, lane 2). This could be consistent with the hypothesis that p55 is a less mature form of p80, generated by proteolysis and/or post translational processing, and that its affinity for apamin binding may be far lower than that of p80.

In order to localize p80 and p55 in rat tissue sections, the technique of immunoperoxidase is employed. To this end, formalin fixed paraffin embedded tissue sections are incubated with the ascitic fluid containing the monoclonal antibody D157 at a dilution of 1:10. This monoclonal antibody, in common with a large number of other murine monoclonal antibodies raised against cell surface receptors, is nonreactive or weakly reactive with the formaldehyde fixed tissues (see Cornet, W.C., et al., J. Immunol., Methods 84: 321-326 (1985) for an example). Therefore, in this study, it is necessary to use a more concentrated preparation of the antibodies for a consistent staining pattern. As shown in Figure 4 (A and B), in the rat kidney, antibody staining is primarily observed in the macula densa and visceral layer of Bowman's capsule (Figure 4A) and the luminar surfaces of certain distal convoluted tubules (Figure 4B). No staining of these structures are observed in the control sections (Figure 4C). At present, the significance of such a restricted localization of p80 in the kidney is unclear. The appearance of the anti-p80 immunoreactivity in certain distal tubules may reflect the developmental and/or functional stages of those distal tubules. A number of reports have identified calcium-activated K-channels in the rabbit renal brush border membranes (Zweifach, A., et al., Amer. J. Physiol. 261: F187-F196 (1991)), in the apical membrane of cultured collecting duct epithelium (Laskowski, F.H., et al., Renal Phys. Biochem. 13: 70-81 (1990)), in cultured medullary thick ascending limb cells (Cornejo, M., et al., J. Membr. Biol. 110: 49-56 (1989)) and in the luminal membrane of the tubule cells in thick ascending limb of Henle's loop (Klaerke, D.A., et al., J. Membr. Biol. 95: 105-112 (1987)). In the latter location, the K-channel may be required for maintenance of the lumen positive transepithelial potential and may be important for regulation of NaCl reabsorption (Klaerke, D.A., et al., J. Membr. Biol. 95: 105-112 (1987)), for K⁺secretion, and for cell volume regulation (Lu, L., et al., J. Biol. Chem. 265: 16190-16194 (1990) and Wang, W., et al., Annu. Rev. Physiol. 54: 81-96 (1992)). In most cases studied, however, the majority of the Ca-activated K-channel involved in the renal function are of the BK type (Wang, W. et al., Ann. Rev. Psysiol. 54: 81-96 (1992)).

In the rat brain, the monoclonal antibody D157 shows reactivity with the choroid plexus (Figure 4D) and the hippocampal neurons and naked nerve fibers. These structures are not stained in the control brain sections (Figure 4E). Using [¹²⁵I]apamin, Mourre et al., (Brain Res. 382: 239-249 (1986)) studied the distribution of apamin binding sites in various regions of the rat brain. A high density of binding sites was observed in the hippocampal neurons, habenula and olfactory bulb. Another binding study with [¹²⁵I]apamin (Habermann, E. et al., Eur. J. Biochem. 94: 355-364 (1979)) indicated that the binding sites are primarily enriched in the rat forebrain, brain stem and the cerebellum.

The results described herein are consistent with the interpretation of the 80 KDa protein as an apamin receptor associated with a calcium-activated K+ channel. Further analysis of the predicted amino acid sequence, as described below, confirms a structure having four putative hydrophobic transmembrane domains, and a putative calcium binding domain. The latter shows significant homology to a component of a calcium activated K⁺ channel in Drosophila (Atkinson, N.S. et al., Science 253: 551-555 (1991)).

Those skilled in the art will recognize that, although the exemplified apamin binding protein is derived from brain, the invention is not limited to a protein derived from this source. As the immunochemical data presented herein demonstrates, homologous proteins exist in other tissues and other species, and are isolatable by the methods described herein. The invention thus also encompasses those apamin binding proteins produced in other tissues, in particular those expressed in heart, vascular smooth muscle, neurons, kidneys, pancreas, human melanomas and neuroblastomas.

As noted above, the isolated protein, which on SDS-PAGE is about 90-95% pure, is useful for the generation of monoclonal and polyclonal antibodies which are in turn useful in screening DNA libraries for production of apamin binding protein cDNA homologues. Since the apamin receptor is also expressed in melanomas and neuroblastomas, the monoclonal antibodies are useful in tumor imaging when conjugated to an appropriate imaging agent, or in tumor therapy, when conjugated to an appropriate cytotoxic agent. Also, the monoclonal antibodies are useful for affinity purification of apamin receptors from other tissues. For example, the antibody can be immobilized in the same manner as apamin on EAH-Sepharose, as described in the examples below, and used in the affinity chromatography in substantially the same manner. Alternately, the antibody can be immobilized on cyanogen bromide activated sepharose, Affigel®, or any other appropriate affinity matrix. Such matrices are well known to those skilled in the art.

The isolated receptor protein is also useful in assays for identifying compounds which may act as analogs of apamin, i.e., which can modulate the activity of the apamin receptor. For example, the receptor protein can be immobilized by any means which does not interfere with apamin binding activity. The immobilized receptor is then contacted with a specific compound or mixture and the ability to compete with radiolabelled apamin for binding to the receptor is evaluated. Also, p80 or other isolated apamin binding protein is useful for developing an immunoassay to measure the level of apamin receptor in patients' serum for diagnosis of neural degeneration. Variations of this method will be apparent to those skilled in the art. The above embodiments of the invention are further described in examples 1-6.

A full-length apamin binding protein nucleic acid sequence, presumed to be associated with a calcium activated K⁺ channel, is first isolated from a porcine vascular smooth muscle (aorta) expression cDNA library in a λ-ZAP vector. The library is screened with polyclonal sera raised against a bovine brain apamin receptor. Screening of about 2 million plaque forming units yields four positive plaques which are rescreened and plague purified.

The λZAP is transformed into "pBluescript" plasmid by standard techniques; the DNA is then digested with the restriction endonucleases EcoRI and XhoI to release the cDNA inserts, and analyzed by agarose gel electrophoresis. One 1.6 Kb cDNA clone (designated Kcal 1.6) is selected for Northern hybridization, genomic Southern blotting and DNA sequencing. As shown in Figure 5A, the cDNA Kcal 1.6 detects a single band of approximately 2.1 Kb in the adult rat brain mRNA (lane 1), bovine brain mRNA (lane 2) and porcine brain mRNA (lane 3). The probe, however, reveals two mRNA bands of 2.1 and 3.0 Kb in size in the Northern blot of mRNA from neonatal rat brain (Figure 5B). These results suggest that in the neonatal rat brain, there are two distinct mRNA species which hybridize to Kcal 1.6, possibly arising by the alternate splicing of mRNA.

Next, an EcoRI cut-genomic southern blot probed with Kcal 1.6 cDNA. As shown in Figure 6, after repeated washing of the blot at high stringency, the Kcal 1.6 probe detects a single 14 Kb band in human (lane 1) and in monkey (lane 2). However, there are variable patterns of hybridization in the rat (lane 3), mouse (lane 4), canine (lane 5), bovine (lane 6), rabbit (lane 7) and chicken (lane 8). There is no detectable hybridization with the yeast DNA (lane 9). This experiment indicates that there are significant sequence homologies among the genes encoding p80 in various species. Furthermore, the gene(s) encoding p80 in human and monkey are probably more similar than those in other species.

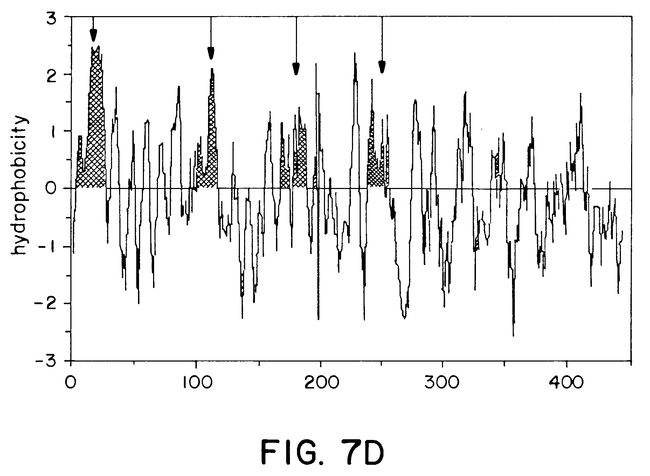

Kcal 1.6 cDNA is then sequenced. The nucleotide sequence obtained indicates that the clone is not of full length and lacks the initiation methionine residue. To obtain a full-length clone, Kcal 1.6 is used as a probe and the original porcine aorta cDNA library is screened, and positive clones analyzed by restriction mapping and electrophoresis for relatedness and insert size. One cDNA clone (designated Kcal 1.8), which appears to be slightly longer than Kcal 1.6, is selected and sequenced by a Taq polymerase sequencing technique. When the nucleotide sequence (SEQ ID No.:1) is translated in frame, the cDNA Kcal 1.8 encodes a protein (SEQ ID No.:2) of 437 amino acids (Figure 7A-7C), with an initiation methionine and a stop site. Hydrophobicity analysis (Figure 7D) of the sequence indicates the presence of four strongly hydrophobic putative transmembrane domains (TMDI-4), a short amino terminus and a long carboxyl terminus. The sequence has some interesting features. It contains a strong "EF-Hand" consensus sequence (in Figure 7A-7C), indicated by an asterisk (*). The EF-Hand consensus sequence is present in virtually all calcium binding protein members of calmodulin and troponin C families. In fact, the EF-Hand motif in Kcal 1.8 almost perfectly matches that of calmodulin, as well as a recently cloned component of Drosophila calcium activated K-channel, "Slo" (Ohandra, M. et al., Toxicol. Pathol. 19: 164-167 (1991)). In addition, the sequence flanking the putative "EF-Hand" motif of Kcal 1.8 has significant homology with a number of known calcium binding proteins including troponin C, myosin, calreticulin, PEP-19, and several others. Since the small conductance calcium-activated potassium channel (sKca) must have a calcium binding site, it gives further support to the belief that Kcal 1.8 indeed encodes sKca. If the "EF-Hand" motif is in fact a calcium binding site of Kcal 1.8 protein, it places the "EF-Hand" motif on the cytoplasmic side of the membrane. The amino acid sequence of Kcal 1.8 also contains one protein kinase C site, and one tyrosine kinase phosphorylation site (not shown). In addition, a "leucine zipper" motif can be identified in the C-terminal portion of the protein (Figure 7A-7C, boxed "L"). At present, the significance, if any, of this motif in Kcal 1.8 is unclear. However, the presence of these putative phosphorylation sites, together with the "EF-Hand" motif are likely to place both N- and C-termini of the protein in the cytoplasmic side of the plasma membrane.

To further confirm Kcal 1.8's identity as an apamin receptor, Kcal cDNA is introduced into a stable mammalian expression vector, pRC/CMV, which is used to transfect CV-I cells (African green monkey kidney). Cells stably expressing the Kcal 1.8 gene product are selected and are contacted with radiolabelled apamin, in the presence or absence of unlabelled apamin. A number of transfectants show enhanced binding of radiolabelled apamin, thereby adding further confirmation of Kcal 1.8's identity.

The foregoing discussion, and the sequences provided in Figure 7A-7C (SEQ ID No.:1 and No.:2), relate to a porcine smooth muscle apamin receptor. However, it will be understood that the invention encompasses more than the specific exemplary sequences. Modifications to the sequence, such as deletions, insertions, or substitutions in the sequence which produce silent changes in the resulting protein molecule are also contemplated. For example, alteration in the gene sequence which reflect the degeneracy of the genetic code, or which result in the production of a chemically equivalent amino acid at a given site, are contemplated; thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a biologically equivalent product. Nucleotide changes which result in alteration of the N-terminal and C-terminal portions of the protein molecule would also not be expected to alter the activity of the protein. It may also be desirable to eliminate one or more of the cysteines present in the sequence, as the presence of cysteines may result in the undesirable formation of multimers when the protein is produced recombinantly, thereby complicating the purification and crystallization processes. In some cases, it may in fact be desirable to make mutants of the sequence in order to study the effect of alteration on the biological activity of the protein. Each of the proposed modifications is well within the routine skill in the art, as is determination of retention of biological activity of the encoded products.

The invention also encompasses homologous sequences obtained from other species and other tissues. As has already been demonstrated above, the nucleic acid sequence depicted in Figure 7A-7C (SEQ ID No.:1) hybridizes, under relatively stringent conditions, with nucleic acid fragments present in a number of other species, including human, thus demonstrating the ability to isolate other non-porcine sequences. Moreover, apamin receptors from tissue types other than vascular smooth muscle are also known to exist. Brain, skeletal muscle, and liver, in addition to vascular smooth muscle, have been repeatedly demonstrated to express a single class of binding site (Haylett, B.G., et al., Potassium Channels: Structure, Classification, Function and Therapeutic Potential, 70-95 (1990); Habermann, E. et al., Eur. J. Biochem. 94: 355-364 (1979); Mourre, C. et al., Brain Res. 382: 239-249 (1986); (Seagar, J.J., et al., Biochemistry 25: 4051-4057 (1986); Seagar, M.J., et al., J. Neurosci. 7: 565-570 (1987); Schmid-Antomarchi, H., et al., Eur. J. Biochem. 142: 1-6 (1984); and Wu, K., et al., Brain Res. 360: 183-194 (1985)). On the other hand, cardiac tissue seems to exhibit a heterogeneous population of target sites. The sequence disclosed in Figure 7A-7C (SEQ ID No.:1) can thus be used as a probe to isolate the corresponding receptors from other species and tissues. Alternate receptor types are isolatable as follows. cDNA libraries prepared from mRNA from the specific tissue type of interest are probed with radiolabelled Kcal 1.8 cDNA and washed under medium stringency (e.g., 1 x SSC, 0.1% SDS, 55°C). Plaques which appear positive are rescreened to verify authenticity. The positive plaques are then used in plasmid rescue according to techniques known in the art. Rescued plasmids are purified, cut with appropriate restriction enzymes, and analyzed in an agarose gel stained with ethidium bromide. The second gel is transferred to an nitrocellulose filter, probed with labelled Kcal 1.8, washed sequentially under a medium, then high stringency (0.1 x SSC, 0.1% SDS, at 65°C) wash and exposed to X-ray film. Those inserts which strongly hybridize to Kcal 1.8 under high stringency conditions represent likely receptor cDNA candidates. Further confirmation of the identity of these putative receptors can be accomplished according to the protocols described in the following examples, or in accordance with routine techniques known in the art. Thus, the invention encompasses not only the nucleotide and amino acid sequences depicted in Figure 7A-7C (SEQ ID No.:1 and No.:2), but also nucleotide sequences which hybridize, under medium or high stringency conditions, with nucleotide sequence (SEQ ID No.:1) encoding the amino acid sequence (SEQ ID No.:2) of Figure 7A-7C, as well as the biologically active proteins or fragments encoded thereby.

The nucleic acid sequence can be used to express the receptor protein in a variety of host cells, both prokaryotic and eukaryotic, for the chosen cell line. Examples of suitable eukaryotic cells include mammalian cells, plant cells, yeast cells, and insect cells. Suitable prokaryotic hosts include Escherichia coli and Bacillus subtilis.

Suitable expression vectors are selected based upon the choice of host cell. Numerous vectors suitable for use in transforming bacterial cells are well known. For example, plasmids and bacteriophages, such as λ phage, are the most commonly used vectors for bacterial hosts, and for E. coli in particular. In both mammalian and insect cells, virus vectors are frequently used to obtain expression of exogenous DNA. In particular, mammalian cells are commonly transformed with SV40, Polyoma virus, or transfected with plasmids such as pRC/CMV; and insect cells in culture may be transformed with baculovirus expression vectors. Yeast vector systems include yeast centromere plasmids, yeast episomal plasmids and yeast integrating plasmids. The invention encompasses any and all host cells transformed or transfected by the claimed nucleic acid fragments, as well as expression vectors used to achieve this. In particular, the host cells chosen for transfection are cells which exhibit only low (i.e., background) levels of receptor expression (e.g., see Figure 8) before transcription.

In a preferred embodiment, nucleic acid sequences encoding an apamin receptor are used to transfect eukaryotic cells, preferably mammalian cells. For an initial determination of the ability of a given sequence to produce an apamin binding protein, transient expression, using plasmids such as pcDNAI or PSG5 into which the putative receptor DNA sequence has been ligated, and CMT-1 or COS-1 or -7 cells, can be employed. CMT-1 cells are transfected using the calcium phosphate precipitation method, and within 24 hours of transfection, the SV40 large T antigen is induced with addition of zinc to the medium. Seventy-two hours after transfection, cells are harvested for either RNA isolation or apamin binding assays. Expression is compared between cDNA and mock-transfected cells to determine if receptor activity is achieved by transfected cells. A positive host cell is preferably one which exhibits about twice the background level of apamin binding observed in non-transfected host cells of the same type.

For use of the sequences in screen development, stable expression of the DNA may be desirable. In this case, the DNA encoding the receptor is ligated into a stable vector containing a selectable marker, such as pRC/CMV, pcDNAI Neo, pXTI, or pMAM Neo. The plasmid DNA is linearized and introduced into an appropriate cell line for such vectors, e.g., CV-1, CHO, HepG-2 or NIH3T3 cells, by electroporation. Successfully transfected cells are identified by selection and isolated clones are picked and amplified. To determine transcription of Kcal message, cellular RNA is isolated and separated electrophoretically on agarose gel. Detection of endogenous and exogenous mRNA is accomplished using Kcal 1.8 as a probe.

Identification of exogenous (transfected) mRNA is accomplished by probing with a 400 bp fragment from the 5' untranslated region of cDNA, since this region is most divergent among species, diminishing the incidence of cross-hybridization.

The ability of any given isolated DNA sequence to yield a functional apamin receptor is determinable by a simple apamin binding assay. Transfected cells are prepared as previously described (Daniel, S et al., J. Pharmacol. Methods 25: 185-193 (1991)). Binding assays are performed by a standard procedure (Mourre, C., et al., Brain Res. 382: 239-249 (1986)), and values for maximum binding of ligand to receptor (Bmax) and dissociation constant (K_{d}) for each cell line is calculated.

Further evaluation of the measurement of potassium channel activity in cultured transfectant cells is accomplished by ⁸⁶Rb efflux assay (Vaitukatis, J.L., Methods in Enzymology 73: 46-52 (1981), incorporated herein by reference). Briefly, stably transfected cells are loaded overnight with ⁸⁶Rb in microtiter plates, the medium is then discarded and adherent cells washed three times to remove isotope. Cells are then incubated for 30 minutes at 37°C with an isotonic buffer containing 20 mM CaCl₂ and 100 µM calcium ionophore A23187. The supernatants from wells are recovered and counted. The cell layer is solubilized in Triton X-100 and also counted, and the percent efflux of ⁸⁶Rb calculated as described. The experiment is carried out in the presence or absence of 1 mM apamin (an sKca blocker) or 1 µM charybdotoxin (a BKca blocker), and control experiments carried out in parallel with cells being incubated with buffer, but without added ionophore. The percent efflux in transfectants harboring cloned DNA mock transfectants, and wild-type CV-I cells (to measure endogenous efflux) are compared. Such assays are also useful in determining the effect of structural change in the channel in its function, and also to evaluate functional differences between different receptor subtypes. This assay is useful both in confirming activity of a putative receptor/channel as well as confirming the effects.

### Deposit of Biological Materials

The following biological materials were deposited with the American Type Culture Collection, 12301 parklawn Drive, Rockville, Maryland, on June 15, 1992, and given the Accession Numbers indicated:

| Material | Accession No. |
|---|---|
| E. coli containing pBluescript plasmid containing Kcal 1.8 | ATCC 69017 |

The above embodiments of the current invention are further illustrated by examples 7-9.

### EXAMPLES

### 1. Tissue Homogenization and Plasma Membrane Solubilization

Freshly frozen bovine brain (300 g) is homogenized for two minutes in a Waring blender in five volumes of buffer "H"; tris-HC1 (20 mM), KCl (3.0 mM), phenylmethylsulfonylfluoride, PSMF (0.1 mM), EDTA (0.1 mM), leupeptin (2.0 ug/ml), pH 7.0 at 4°C. The homogenate is centrifuged at 500 x g for 45 minutes at 4°C. The supernatant is discharged, the crude membrane pellet is resuspended in 100 ml of buffer "H" containing 15% v/v glycerol and stored in 10 ml aliquots at -80°C until used.

In order to determine which detergent permits the binding of the apamin binding proteins to this affinity matrix, a number of ionic and non-ionic detergents are tested. The maximum degree of binding is observed when 1% w/v CHAPS or Lubrol-PX is used to solubilize the synaptosomes. A 1% v/v preparation of Triton X-I00, Nonidet P-40, or SDS does not permit an efficient binding of the solid phase apamin to its binding proteins. In view of the dialysable nature of CHAPS, it is selected as the solubilizing detergent.

Frozen aliquots (50 ml) are rapidly thawed in a 37°C water bath and gently mixed with an equal volume of ice-cold buffer "S": tris-HCl (40 mM), KC1 (10 mM), CaC12 (0.1 mM), MgC1₂ (0.1 mM) and CHAPS (2% w/v), pH 7.4. The mixture is shaken gently at 4°C for 30 minutes, centrifuged at 30,000 x g for 30 minutes at 4° and the clear supernatant is collected.

### 2. Preparation of Apamin-Sepharose 4B Affinity Matrix

EAH-Sepharose 4B (20 ml, Pharmacia, LKB, Piscataway, NJ) is washed with 20 mM MOPS (4-morpholine propane sulfonic acid), pH 7.0 as described by the manufacturer. The beads are resuspended in 5.0 ml of 20 mM, MOPS, pH 7.0 containing 0.5 µmol of apamin and supplemented with 1.0 pmol of [¹²⁵I)apamin as a tracer. Solid l-ethyl-3-(3-dimethylaminopropyl) carbodiimide hydrochloride (EDC, Pierce Chemical Co., Rockford, IL) and N-hydroxysulfosuccinimide (sulfo- NHS, Pierce Chemical Co., Rockford, IL) are added to the suspension at a final concentration of 100 mM and 5.0 mM, respectively. The suspension is mixed for 24 hours at 4°C, the beads are washed three times with 10 volumes of 20 mM, MOPS, pH 7.0 containing 0.5M NaCl and an aliquot is counted in a gamma counter. The coupling efficiency is calculated based on the coupling of the tracer apamin. The method results in the coupling of 33 nmole of apamin per milliliter of the EAH Sepharose beads.

### 3. Purification of Apamin Binding Proteins

The CHAPS solubilized membrane (50 ml) is pre-cleared twice, each for 30 minutes at 4°C by incubation and shaking with 1.0 ml of EAH-Sepharose beads (packed volume). The beads are removed by centrifugation at 500 x g for 5 minutes and the supernatants are added to 1.0 ml of apamin-Sepharose beads (packed volume). The suspension is incubated at 4°C for 4 hours with constant shaking, centrifuged at 500 x g for 5 minutes at 4° and the supernatant discarded. The beads are washed four times at 4°C, each time by gently resuspending in 50 ml of ice-cold buffer "S" diluted with an equal volume of distilled water, followed by centrifugation as above. In order to elute the apamin binding proteins, the final pellet of beads is resuspended in 10 ml of tris-HCl (10 mM), KCl (10 mM), pH 7.4 (binding buffer, "B") containing CHAPS (0.1% w/v) and placed in a 42°C water bath for 15 minutes with constant shaking. As shown in Figure 1, incubation of the beads in elution buffer for 15 minutes at 37°C does not lead to elution, but the rapid shift to 42°C results in elution of an 80 KD protein, p80 (Figure 1, lane 1). Further incubation at 55°C does not result in detectable elution of other proteins (Figure 3, lane 3). The beads are removed by centrifugation at 1500 x g for 10 minutes and the supernatant is collected. This procedure is repeated once more and the supernatant is collected. The supernatants containing the eluted apamin binding protein are either used immediately for binding studies, or concentrated by negative pressure dialysis at 4°C and stored at -20°C.

### 4. Cross-Linking of [¹²⁵I]Apamin Binding Proteins

The specificity of the interaction between p80 and apamin is tested by a cross-linking experiment. The protein fraction (100 ng) eluted from the apamin affinity matrix is incubated on ice for 1 hour with 1.0 pmol of [¹²⁵I]apamin (2200 Ci/mmol, New England Nuclear, Boston, MA), in a final volume of 100 µ1 of buffer "B", with or without the inclusion of unlabelled apamin (final concentration of 1.0 µM). This is an essential precaution since the apamin binding ability of p80 is lost within 2 hours of elution from the affinity matrix. This observation of instability of detergent solubilized apamin binding proteins is in agreement with the findings of Schmid-Antomarchi et al. (Eur. J. Biochem. 142: 1-6 (1984)). The [¹²⁵I]apamin-P80 complex is then separated from unbound [¹²⁵I]apamin by loading at 4°C on a G-50 Sephadex "Quickspin" column (Boehringer Mannheim, Indianapolis, IN) pre-equilibrated with sodium borate (50 mM) buffer, pH 8.0. To the collected void fraction (100 µ1) is added 10 µ1 of a 10 mM stock in dimethylsulfoxide of dimethylsuberimidate (DMS, Pierce Chemical Co., Rockford, IL). The mixture is incubated on ice for 1 hour, quenched by addition of ammonium acetate (100 mM final), lyophilized, counted in a gamma counter and analyzed by SDS-PAGE using a Phast System (LKB-Pharmacia, Piscataway, NJ) or by using conventional techniques. Gels are fixed and stained with 0.1% Phase Gel-Blue (LKB-Pharmacia), 20% acetic acid and 20% v/v methanol and destained in 5% v/v acetic acid and 25% v/v methanol in distilled water. Autoradiography is carried out at -80°C using X-OMAT-AR films and one intensifying screen.

As shown in Figure 2 (lane 1), [¹²⁵I]apamin is uniquely cross-linked to p80, while the excess and uncross-linked [¹²⁵I]apamin appears at the dye front. The interaction is considered specific, since the inclusion of the excess unlabeled apamin completely abrogates the cross-linking of [¹²⁵I]apamin to p80 (Figure 2, lane 2). In addition to DMS, the same experiment is repeated with four other, chemically distinct, bifunctional cross-linking reagents. Each time, the cross-linked protein band on the autoradiogram is superimposable to the p80 band in the Coomassie stained gel.

### 5. Preparation of Monoclonal Antibodies

Female balb/c mice (8-10 weeks old) are immunized with the affinity purified apamin binding protein as prepared above. Mice are immunized with a total of 2.0 µg of protein emulsified in complete Freund's adjuvant by one intraperitoneal and six equal subcutaneous injections. Animals are rested for 30 days, following which they are immunized as before, but with incomplete Freund's adjuvant. The animals are boosted every two weeks by a single intraperitoneal injection of 100 ng of the protein emulsified in incomplete Freund's adjuvant. Fourteen days after the fourth immunization, mice are test bled from the orbital sinus and polyclonal sera collected and tested for reaction with affinity purified receptor. A final immunization is then given, and three days later, the animals are sacrificed and the spleens removed.

Splenocytes from animals producing positive polyclonal sera are fused with the murine myeloma cell line X63-Ag 8.653. Hybridomas are selected and subcloned by standard procedures (Ausuber, F.M., et al., (eds.), Current Protocols In Molecular Biology II: 11.3-11.16, Wiley Interscience (1989)). Hybridoma supernatants are screened in a solid phase ELISA using purified apamin binding protein as the target antigen as previously described (Zia, M.R., et al., Immunol. Methods 82: 233-241 (1985) and Hayashibe, K., et al., J. Immunoassay 11: 89-95 (1990)), as well as in an apamin binding neutralization assay. In this assay, bovine brain plasma membranes are incubated with the diluted hybridoma for 1 hour at 4°C. The binding assay is carried out at 4°C in buffer "B": tris-HCl (20 mM), KCl (5.0 mM), BSA (0.1% w/v), PMSF (0.1 mM), SCH 32,615 (0.1 mM), pH 7.4. A binding assay for receptor is performed in a total volume of 200 µ1; synaptosomal membranes (50 µ1), [¹²⁵I]apamin (0.1 pmol in 20 µ), hybridoma supernatants (50 µ1) and buffer B (to 200 µ1). The mixture is incubated at 4°C for 2 hours. The assay mixtures are filtered on a glass fiber filter GF/C. The membranes are washed with buffer B and filters are counted in a Pharmacia-LKB gamma counter. Acitic fluids are produced and collected in balb/c mice pretreated with pristane using standard protocols. The foregoing procedure yields at least two positive hybridomas, producing monoclonal antibodies identified herein as A114 and D157. Both antibodies are of the IgG1 subtype, with no detectable contamination with any other IgG or IgM isotypes.

### 6. Distribution of p80

Polyclonal and monoclonal antibodies are generated to p80 to enable characterization of p80 Western blotting and to perform immunocytochemistry in tissue sections. The anti-p80 polyclonal antisera from two immunized mice are used in Western blotting (standard techniques, detected by Vectastain ABC Kit, Vector Laboratories, Burlingame, CA) using a rat brain synaptosomal preparation separated on SDS-PAGE, as described above, and transferred onto nitrocellulose. As shown in Figure 3(A), the antisera from two immunized mice react with a major 80 KDa protein (p80) and a minor 55 KDa protein (p55) bands (lanes 1 and 2), neither of which is detected when a pre-immune serum is used (lane 3). The presence of an immunochemical reactivity towards a p55 protein suggests two possibilities. Either the murine antiserum is not mono-specific, or the p80 and p55 are immunologically related. To resolve this question, using the monoclonal antibody D157, the p80/p55 profile is analyzed in several rat tissues. Preparation of the formalin fixed, paraffin embedded sections from rat tissues, application of primary antibodies, detection by the avidin-horseradish peroxidase (Dako), counter shaking by hematoxylin and mounting are performed by standard procedures and as described (Ohandra, M. et al., Toxicol. Pathol. 19: 164-167 (1991)). As shown in Figure 3(B), the monoclonal antibody D157, diluted 1:10,000, reacts strongly with p80 and p55 in membranes isolated from bovine brain (lane 1), rat brain (lane 2), rat heart (lane 3), rat kidney (lane 4) and rat liver (lane 5). In the rat kidney and liver, the p55 band appears as a doublet (Figure 3B, lanes 4 and 5). Identical results are obtained with the other anti-p80 monoclonal antibody, A114 at 1:10,000 dilution. The immunoreactivity of these monoclonal antibodies with p80 and p55 indicates that the two proteins must be immunologically related. The presence of a 55 KDa protein, in addition to a 80 KDa apamin binding protein, has been documented by several groups (Seagar, M.J., et al., Biochemistry 25: 4051-4057 (1986), Seagar, M.J., et al., J. Biol. Chem. 260: 3895-3898 (1985), Leveque, C., et al., FEBS Letters 275: 185-189 (1990) and Marqueze, B., et al., Eur. J. Biochem 169: 295-298 (1987)).

### 7. Screening Expression Library.

A porcine aorta expression cDNA library in λ-Uni ZAP λR (Stratagene, La Jolla, CA) is probed with a 1:1000 dilution of a murine anti-apamin binding protein polyclonal antiserum (M2) using the Vectastain ABC kit (Vector Laboratories Inc., Burlingame, CA) as the secondary antibody and detection system. Approximately 2 x 10⁶ plaque forming units are screened in this manner.

Four positive plaques are selected from the first round of screening. These are subjected to a re-screen and plasmids (pBluescript) containing the cDNA inserts are rescued using a helper phage. The parent plasmid DNA is digested with the restriction endonucleases EcoRI and XhoI to release the cDNA inserts and analyzed by agarose gel electrophoresis. one 1.6 Kb cDNA clone (designated Kcal 1.6) is selected for Northern hybridization, genomic Southern blotting and DNA sequencing. For Northern hybridization, mRNA is isolated from frozen rat tissues using "Fast Track" mRNA isolation kit (Invitrogen, San Diego, CA) or purchased from Clontech Labs (Palo Alto, CA). Genomic southern blot, "Zoo-blot" is purchased from Clontech Labs and processed as described by the manufacturer. As shown in Figure 5A, the cDNA Kcal 1.6 detects a single mRNA band of approximately 2.1 Kb in the adult rat brain mRNA (lane 1) bovine brain mRNA (lane 2) and porcine brain mRNA (lane 3). The probe, however, reveals two mRNA bands of 2.1 and 3.0 Kb in size in the northern blot of mRNA from neonatal rat brain (Figure 5B). These results may indicate that in the neonatal rat brain, there are two distinct mRNA species which hybridize to Kcal 1.6, possibly arising from the alternate splicing of mRNA. Next, an EcoRI cut-genomic southern blot is probed with Kcal 1.6 cDNA. As shown in Figure 6, after repeated washing of the blot at high stringency, the Kcal 1.6 probe detects a single 14 Kb band in human (lane 1) and in monkey (lane 2). However, there are variable patterns of hybridization in the rat (lane 3), mouse (lane 4), canine (lane 5), bovine (lane 6), rabbit (lane 7) and chicken (lane 8) ranging from 14 Kb to 3.0 Kb. There is no detectable hybridization with the yeast DNA (lane 9). These results indicate that there are notable homologies among the genes encoding p80 in various species.

### 8. Sequencing of Kcal 1.6

DNA sequencing is performed using the "Taq-Track" sequencing system (Promega Corp.) or the "Sequenase" system (U.S. Biochemical, Cleveland, OH). The nucleotide sequence obtained indicates that the clone is not full length, and lacks an initiation methionine residue. To obtain a full-length clone, Kcal 1.6 is used as a probe to screen the original porcine aorta cDNA library. Positive clones are analyzed by restriction mapping and electrophoresis for relatedness and insert size. One cDNA clone (designated Kcal 1.8) which is slightly longer than Kcal 1.6 is isolated and sequenced. The nucleotide and amino acid sequence of Kcal 1.8 is shown in Figure 7A-7C. The cDNA encodes 437 amino acids; the hydropathy plot (Figure 7D) indicates four strongly hydrophobic putative transmembrane domains. There is a putative calcium binding domain which closely matches that of the cloned cDNA slo encoding a putative calcium activated K-channel in Drosophila. However, there is no significant sequence homology between Kcal 1.8 and slo in other regions.

There is one strong consensus sequence in Kcal 1.8 for the cAMP dependent protein kinase, as well as those putative casein kinase phosphorylation sites. The Kcal 1.8 sequence has no significant homologies with any known voltage gated K-channels, sodium channels or calcium channels.

### 9. Expression of Kcal 1.8

CV-1 cells (ATCC CCL70) stably expressing the Kcal 1.8 gene product are produced by introducing the cDNA in the stable mammalian expression plasmid, pRc/CMV (InVitrogen) which contains a Neo^{r}marker. The Kcal 1.8 sequence is extracted from the pBluescript vector by digestion, with EcoRI and XhoI, and ligated into the corresponding sites of pRc/CMV. To transfect the cells, confluent 100 mm dishes of CV-1 cells are split and replated the day before the transfection, to ensure the cells are in log-growth phase. For electroporation, cells are harvested with trypsin, washed once with phosphate-buffered saline, and twice with an isotonic, low ionic strength buffer containing 272 mM sucrose, 7 mM sodium phosphate, pH 7.4 and 1 mM MgCl₂ (buffer E). The cells are resuspended in this same buffer to a final concentration of 1.5 x 10⁶ cells/ml. Twenty µg of the appropriate vector are digested with 40 units of ScaI for 2 hours at 37°C to linearize the plasmid. The linearized plasmid is phenol/chloroform extracted, EtOH precipitated, and resuspended in 400 µl of Buffer E. The resuspended DNA is mixed with 400 ul of CV-1 cells (1 x 10⁶ cells) and incubated at room temperature for 2 minutes prior to electroporation. Electroporation is accomplished using a Bio-Rad gene pulser with a 300-V pulse at 25 µFarads. Transfections are done in duplicate. The suspension is allowed to further incubate for 5 minutes at room temperature, and then plated onto 100 mm tissue culture dishes with 10 mls of Dulbecco's modified Eagle's medium containing 10% fetal calf serum. Two days following transfection, G418 is added to a final concentration of 200 ug/ml. Isolated G418-resistant colonies are identified. They are selected with cloning cylinders and amplified.

Transfected cells are harvested and washed. They are incubated with [¹²⁵I]apamin in the binding buffer "B": Tris-HCl 10mM, KCl 10 mM, pH 7.4, in the presence or absence of 1 uM cold apamin. The incubation is at 4°C for 30 minutes with cold apamin, followed by 1 hour incubation at 4°C with [¹²⁵I]apamin (20,000 cpm/well). Target cells are then filtered and washed with the binding buffer plus BSA. The filters are counted in a gamma counter.

As shown in Figure 8, Transfectant #1, 3, 10 and 12 show significantly enhanced binding of [¹²⁵I]apamin, compared to other transfectants shown.

## Claims

1. A composition of matter useful as an affinity matrix for isolating an apamin receptor from cellular material comprising a solid support matrix having a free amino group covalently coupled to a free carboxyl group of apamin.

2. The composition of Claim l in which the solid support matrix comprises a derivatized agarose matrix comprising a spacer arm with a free amino group.

3. The composition of Claim 2 in which the agarose is EAH-Sepharose.

4. The composition of Claim 1 in the coupling is achieved by carbodiimide condensation.

5. A method of making a composition useful for isolation of an apamin receptor from cellular material comprising contacting a derivatized agarose matrix having a free amino group with apamin in the presence of a carbodiimide under condensation reaction conditions and for a time sufficient to permit coupling of the matrix and apamin.

6. The method of Claim 5 in which the reaction is conducted at about 4°C.

7. The method of Claim 5 in which the reaction is conducted in the presence of N-hydroxysulfosuccinimide.

8. The method of Claim 5 in which the agarose matrix comprises a spacer arm with a free amino group.

9. The method of Claim 8 in which the matrix is EAH-Sepharose.

10. A method for isolating an apamin receptor from cellular material comprising contacting detergent solubilized cellular material containing an apamin receptor with an affinity matrix comprising a derivatized agarose matrix having a free amino group covalently coupled to apamin, at a maximum temperature of about 4°C, and for a time sufficient to permit binding of receptor to apamin, and eluting receptor from the affinity matrix by raising the temperature to at least about 42°C.

11. The method of Claim 10 in which the agarose matrix comprises a spacer arm having a free amino group.

12. The method of Claim 10 in which the affinity matrix comprises EAH-Sepharose.

13. An isolated and purified protein which specifically binds apamin.

14. The protein of Claim 13 which is isolated from a tissue selected from the group consisting of brain, heart, vascular smooth muscle, kidney, neuron, pancreas, melanoma and neuroblastoma.

15. The protein of Claim 14 which is isolated from brain tissue.

16. The protein of Claim 13 which is about 90-95% pure.

17. The protein of Claim 15 which has a molecular weight of about 80 KDa.

18. The protein of claim 17 which has a molecular weight of about 55 KDa.

19. The protein of Claim 13 which is associated with a small conductance calcium activated potassium channel.

20. An immunogenic composition comprising an effective amount of the composition of Claim 13.

21. A monoclonal antibody which binds to an apamin receptor or portion thereof.

22. The antibody of Claim 9 which is raised against an apamin binding protein having a molecular weight of about 80 kDa.

23. The antibody of Claim 21 conjugated to a cytotoxic agent.

24. The antibody of Claim 21 conjugated to an imaging agent.

25. An isolated nucleic acid fragment comprising a nucleic acid sequence encoding an apamin receptor protein, or biologically active fragment thereof.

26. The fragment of Claim 25 encoding a receptor expressed in a tissue selected from the group consisting of brain, heart, smooth muscle, kidney, liver, pancreas and neuron.

27. The fragment of Claim 25 in which the sequence hybridizes under high stringency conditions, with a nucleic acid sequence encoding the amino acid sequence of SEQ ID No.:2.

28. The fragment of Claim 25 which comprises the sequence of Kcal 1.8.

29. An isolated recombinantly produced apamin receptor protein, or biologically active fragment thereof.

30. A recombinantly produced apamin receptor protein expressed in a host cell which normally expresses only low levels of an apamin receptor.

31. A host cell transformed or transfected with the derivatives fragment of Claim 25, and progeny or thereof.

32. The host cell of Claim 31 which binds apamin at about twice the level of the host cell in its untransformed or untransfected state, and progeny and derivatives thereof.

33. A recombinant vector comprising the fragment of Claim 25.

34. The vector of Claim 10 comprising the sequence depicted in Figure 7 or biologically active fragment thereof.

35. The vector of Claim 34 which comprises sequence of Kcal 1.8.

36. A host cell comprising the vector of Claim 34.

37. A host cell comprising the vector of Claim 35.

38. A method of expressing an apamin receptor in a host cell which comprises transforming or transfecting the host cell with the fragment of Claim 25.

39. The method of Claim 38 in which comprises the nucleic acid sequence depicted in SEQ ID No.:1.

40. The method of Claim 38 in which the fragment comprises the nucleic acid sequence of Kcal 1.8.

41. A method for identifying a compound which is capable of modulating apamin receptor activity comprising contacting a compound or mixture of compounds with a host cell transformed or transfected with the fragment of Claim 25, and observing the presence or absence of an effect on the host cell's ability to bind apamin.

42. A method for identifying a compound which is capable of modulating channel activity which comprises contacting a compound or a mixture of compounds with a host cell transformed or transfected with the fragment of Claim 1, and observing the presence or absence of an effect on the host cell's ability to transport in a ⁸⁶RB efflux assay.


SEQUENCE LISTING

(1) GENERAL INFORMATION:

(i) APPLICANT: American Cyanamid Company

(ii) TITLE OF INVENTION: Composition for Isolating Apamin
Receptors, Apamin Binding Protein, and Uses Therefor

(iii) NUMBER OF SEQUENCES: 2

(iv) CORRESPONDENCE ADDRESS:
(A) ADDRESSEE: Hamilton, Brook, Smith & Reynolds, P.C.
(B) STREET: 2 Militia Drive
(C) CITY: Lexington
(D) STATE: MA
(E) COUNTRY: US
(F) ZIP: 02173

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER:
(B) FILING DATE:
(C) CLASSIFICATION:

(viii) ATTORNEY/AGENT INFORMATION:
(A) NAME: Carroll, Alice O.
(B) REGISTRATION NUMBER: 33,542
(C) REFERENCE/DOCKET NUMBER: ACC92-09M

(ix) TELECOMMUNICATION INFORMATION:
(A) TELEPHONE: 617-861-6240
(B) TELEFAX: 617-861-9540

(2) INFORMATION FOR SEQ ID NO:1:

(i) SEQUENCE CHARACTERISTICS:
(A) LENGTH: 1730 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 140..1456

(xi) SEQUENCE DESCRIPTION: SEQ ID NO:1:

AGCAGCTCCA TAGGCCCAGC CCCGGCGTAC AAGGATCACT TCCGGTGGTA CTTCACTACC 60

AAGAAGCTGC GATTGGGCGA GCGTGGAAGG GGCATTTCCG GTGTCCACCT GCTTGGGTTC 120

TTTGGACAGA AGTAGGAAG ATG GAG CTC GGC GCC GCG GCC CGT GCT TGG TCG 172
Met Glu Leu Gly Ala Ala Ala Arg Ala Trp Ser
1               5                   10

CTC TTG TGG CTG CTG CTT CCC TTG CTT GGC CTG GTC GGC GCC AGC GGT 220
Leu Leu Trp Leu Leu Leu Pro Leu Leu Gly Leu Val Gly Ala Ser Gly
            15                  20                  25

CCC CGT ACC TTA GTG CTT CTG GAC AAC CTC AAC CTG CGG GAG ACG CAT 268
Pro Arg Thr Leu Val Leu Leu Asp Asn Leu Asn Leu Arg Glu Thr His
        30                  35                  40

TCA CTT TTC TTC CGG AGC CTA AAG GAT CGG GGC TTC GTA CTC ACA TTC 316
Ser Leu Phe Phe Arg Ser Leu Lys Asp Arg Gly Phe Val Leu Thr Phe
    45                  50                  55

AAG ACC GCA GAT GAC CCC AGC CTG TCC CTG ATT AAG TAC GGA GAG TTC 364
Lys Thr Ala Asp Asp Pro Ser Leu Ser Leu Ile Lys Tyr Gly Glu Phe
60                  65                  70                  75

CTC TAT GAC AAT CTC ATC GTC TTT TCA CCT TCG GTA GAA GAT TTT GGA 412

Leu Tyr Asp Asn Leu Ile Val Phe Ser Pro Ser Val Glu Asp Phe Gly
                80                  85                  90

GGA AAT ATC AAC GTG GAG ACC ATC AGT ACC TTT ATC GAC GGC GGA GGC 460
Gly Asn Ile Asn Val Glu Thr Ile Ser Thr Phe Ile Asp Gly Gly Gly
            95                  100                 105

AGT GTC CTG GTA GCT GCC AGC TCA GAC ATC GGT GAC CCT CTC CGC GAG 508
Ser Val Leu Val Ala Ala Ser Ser Asp Ile Gly Asp Pro Leu Arg Glu
        110                 115                 120

CTG GGC AGT GAG TGT GGG ATT GAG TTT GAC GAG GAG AAA ACG GCC GTC 556
Leu Gly Ser Glu Cys Gly Ile Glu Phe Asp Glu Glu Lys Thr Ala Val
    125                 130                 135

ATT GAC CAT CAC AAC TAT GAT GTC TCA GAC CTC GGC CAG CAC ACG CTC 604
Ile Asp His His Asn Tyr Asp Val Ser Asp Leu Gly Gln His Thr Leu
140                 145                 150                 155

ATT GTG GCC GAC ACT GAG AAC CTG CTG AAG GCC CCG ACC ATC GTC GGG 652
Ile Val Ala Asp Thr Glu Asn Leu Leu Lys Ala Pro Thr Ile Val Gly
                160                 165                 170

AAG TCA TCT CTG AAT CCC ATC CTC TTC CGA GGT GTT GGG ATG GTG GCT 700
Lys Ser Ser Leu Asn Pro Ile Leu Phe Arg Gly Val Gly Met Val Ala
            175                 180                 185

GAT CCT GAC AAT CCT TTG GTG CTG GAC ATC CTG ACC GGC TCT TCT ACC 748
Asp Pro Asp Asn Pro Leu Val Leu Asp Ile Leu Thr Gly Ser Ser Thr
        190                 195                 200

TCT TAC TCC TTC TTC CCA GAT AAA CCC ATC ACG CAG TAC CCG CAC GCG 796
Ser Tyr Ser Phe Phe Pro Asp Lys Pro Ile Thr Gln Tyr Pro His Ala
    205                 210                 215

GTG GGG AAG AAC ACG CTG CTC ATC GCG GGG CTG CAG GCC CGG AAC AAC 844
Val Gly Lys Asn Thr Leu Leu Ile Ala Gly Leu Gln Ala Arg Asn Asn
220                 225                 230                 235

GCC CGT GTC ATC TTC AGC GGC TCC CTC GAC TTC TTC AGC GAT GCC TTC 892

Ala Arg Val Ile Phe Ser Gly Ser Leu Asp Phe Phe Ser Asp Ala Phe
                240                 245                 250

TTC AAC TCC GCG GTG CAG AAG GCC ACG CCT GGC TCC CAG AGG TAT CCC 940
Phe Asn Ser Ala Val Gln Lys Ala Thr Pro Gly Ser Gln Arg Tyr Pro
            255                 260                 265

CAG ACA GGC AAC TAT GAG CTC GCC GTG GCC CTC TCC CGC TGG GTG TTC 988
Gln Thr Gly Asn Tyr Glu Leu Ala Val Ala Leu Ser Arg Trp Val Phe
        270                 275                 280

AAG GAG GAG GGT GTC CTC CGA GTG GGG CCT GTG TCC CAC CAT CGG GTG 1036
Lys Glu Glu Gly Val Leu Arg Val Gly Pro Val Ser His His Arg Val
    285                 290                 295

GGC GAG AAA GCC CCA CCC AAC GCC TAC ACC GTC ACT GAC CTA GTC GAG 1084
Gly Glu Lys Ala Pro Pro Asn Ala Tyr Thr Val Thr Asp Leu Val Glu
300                 305                 310                 315

TAC AGC ATC GTG ATT GAG CAG CTC TCA CAG GGC AGA TGG GTC CCC TTT 1132
Tyr Ser Ile Val Ile Glu Gln Leu Ser Gln Gly Arg Trp Val Pro Phe
                320                 325                 330

GAT GGC GAC GAC ATT CAG CTG GAG TTT GTC CGC ATC GAT CCT TTC GTG 1180
Asp Gly Asp Asp Ile Gln Leu Glu Phe Val Arg Ile Asp Pro Phe Val
            335                 340                 345

AGG ACC TTC TTG AAG AGG AAA GGC GGC AAG TAC AGC GTC CAG TTC AAG 1228
Arg Thr Phe Leu Lys Arg Lys Gly Gly Lys Tyr Ser Val Gln Phe Lys
        350                 355                 360

TTG CCG GAC GTG TAC GGC GTG TTC CAG TTC AAA GTG GAC TAC AAC CGG 1276
Leu Pro Asp Val Tyr Gly Val Phe Gln Phe Lys Val Asp Tyr Asn Arg
    365                 370                 375

CTG GGC TAC ACG CAC CTG TAC TCC TCC ACT CAG GTG TCC GTG CGG CCC 1324
Leu Gly Tyr Thr His Leu Tyr Ser Ser Thr Gln Val Ser Val Arg Pro
380                 385                 390                 395

CTG CAG GCA CAC GCA GTA CGA GCG CTT CAT CCC CTC GGC TTA CCC CTA 1372

Leu Gln Ala His Ala Val Arg Ala Leu His Pro Leu Gly Leu Pro Leu
                400                 405                 410

CTA CGC CAG CGC CTT CTC CAT GAT GGT CGG GCT CTT CAT CTT CAG CGT 1420
Leu Arg Gln Arg Leu Leu His Asp Gly Arg Ala Leu His Leu Gln Arg
            415                 420                 425

CGT CTT CTT GCA CAT GAA GGA GAA GGA GAA GTC TGACTGAGGG GCCGGGCCGG 1473
Arg Leu Leu Ala His Glu Gly Glu Gly Glu Val
        430                 435

GCCCCAGGAC TCCTTACAAC ACACAGGGAG GGTTTTTATA GGCTTGCCTT CCCCCCCCTT 1533

TATGGTGGGC TTTGTTTGTT TTTAAAGCCA CGGACAATGG CACAGCTTAC CTCAGTGGGA 1593

GATGCAAGAT GAGTACCAGG GGGTGGTTAG GAATAATTTC TAAGTTTTTC CACCTTGAAT 1653

GCTGAGTGGT ATTTTTCATA TGTAAAGTCA ACTGATTTCT AAAATAAAAG AAAAACATCA 1713

CCCTCAGAAA AAAAAAA 1730

(2) INFORMATION FOR SEQ ID NO:2:

(i) SEQUENCE CHARACTERISTICS:
(A) LENGTH: 438 amino acids
(B) TYPE: amino acid
(D) TOPOLOGY: linear

(ii) MOLECULE TYPE: protein

(xi) SEQUENCE DESCRIPTION: SEQ ID NO:2:

Met Glu Leu Gly Ala Ala Ala Arg Ala Trp Ser Leu Leu Trp Leu Leu
1               5                   10                  15

Leu Pro Leu Leu Gly Leu Val Gly Ala Ser Gly Pro Arg Thr Leu Val
            20                  25                  30

Leu Leu Asp Asn Leu Asn Leu Arg Glu Thr His Ser Leu Phe Phe Arg
        35                  40                  45

Ser Leu Lys Asp Arg Gly Phe Val Leu Thr Phe Lys Thr Ala Asp Asp
    50                  55                  60

Pro Ser Leu Ser Leu Ile Lys Tyr Gly Glu Phe Leu Tyr Asp Asn Leu
65                  70                  75                  80

Ile Val Phe Ser Pro Ser Val Glu Asp Phe Gly Gly Asn Ile Asn Val
                85                  90                  95

Glu Thr Ile Ser Thr Phe Ile Asp Gly Gly Gly Ser Val Leu Val Ala
            100                 105                 110

Ala Ser Ser Asp Ile Gly Asp Pro Leu Arg Glu Leu Gly Ser Glu Cys
        115                 120                 125

Gly Ile Glu Phe Asp Glu Glu Lys Thr Ala Val Ile Asp His His Asn
    130                 135                 140

Tyr Asp Val Ser Asp Leu Gly Gln His Thr Leu Ile Val Ala Asp Thr
145                 150                 155                 160

Glu Asn Leu Leu Lys Ala Pro Thr Ile Val Gly Lys Ser Ser Leu Asn
                165                 170                 175

Pro Ile Leu Phe Arg Gly Val Gly Met Val Ala Asp Pro Asp Asn Pro
            180                 185                 190

Leu Val Leu Asp Ile Leu Thr Gly Ser Ser Thr Ser Tyr Ser Phe Phe
        195                 200                 205

Pro Asp Lys Pro Ile Thr Gln Tyr Pro His Ala Val Gly Lys Asn Thr
    210                 215                 220

Leu Leu Ile Ala Gly Leu Gln Ala Arg Asn Asn Ala Arg Val Ile Phe
225                 230                 235                 240

Ser Gly Ser Leu Asp Phe Phe Ser Asp Ala Phe Phe Asn Ser Ala Val
                245                 250                 255

Gln Lys Ala Thr Pro Gly Ser Gln Arg Tyr Pro Gln Thr Gly Asn Tyr
            260                 265                 270

Glu Leu Ala Val Ala Leu Ser Arg Trp Val Phe Lys Glu Glu Gly Val
        275                 280                 285

Leu Arg Val Gly Pro Val Ser His His Arg Val Gly Glu Lys Ala Pro
    290                 295                 300

Pro Asn Ala Tyr Thr Val Thr Asp Leu Val Glu Tyr Ser Ile Val Ile
305                 310                 315                 320

Glu Gln Leu Ser Gln Gly Arg Trp Val Pro Phe Asp Gly Asp Asp Ile
                325                 330                 335

Gln Leu Glu Phe Val Arg Ile Asp Pro Phe Val Arg Thr Phe Leu Lys
            340                 345                 350

Arg Lys Gly Gly Lys Tyr Ser Val Gln Phe Lys Leu Pro Asp Val Tyr
        355                 360                 365

Gly Val Phe Gln Phe Lys Val Asp Tyr Asn Arg Leu Gly Tyr Thr His
    370                 375                 380

Leu Tyr Ser Ser Thr Gln Val Ser Val Arg Pro Leu Gln Ala His Ala
385                 390                 395                 400

Val Arg Ala Leu His Pro Leu Gly Leu Pro Leu Leu Arg Gln Arg Leu
                405                 410                 415

Leu His Asp Gly Arg Ala Leu His Leu Gln Arg Arg Leu Leu Ala His
            420                 425                 430

Glu Gly Glu Gly Glu Val
        435

SEQUENCE LISTING

(1) GENERAL INFORMATION:

(i) APPLICANT:
(A) NAME: American Cyanamid Company
(B) STREET: 1937 West Main Street
(C) CITY: Stamford
(D) STATE: Connecticut
(E) COUNTRY: U.S.A
(F) POSTAL CODE (ZIP): 06904-0060

(ii) TITLE OF INVENTION: Composition for Isolating Apamin Receptors,
Apamin Binding Protein, and Uses Therefor

(iii) NUMBER OF SEQUENCES: 2

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25 (EPO)

(v) CURRENT APPLICATION DATA:
APPLICATION NUMBER: EP 93112165

(2) INFORMATION FOR SEQ ID NO:1:

(i) SEQUENCE CHARACTERISTICS:
(A) LENGTH: 1730 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION: 140..1456

(xi) SEQUENCE DESCRIPTION: SEQ ID NO:1:

AGCAGCTCCA TAGGCCCAGC CCCGGCGTAC AAGGATCACT TCCGGTGGTA CTTCACTACC 60

AAGAAGCTGC GATTGGGCGA GCGTGGAAGG GGCATTTCCG GTGTCCACCT GCTTGGGTTC 120

TTTGGACAGA AGTAGGAAG ATG GAG CTC GGC GCC GCG GCC CGT GCT TGG TCG 172
Met Glu Leu Gly Ala Ala Ala Arg Ala Trp Ser
1               5                   10

CTC TTG TGG CTG CTG CTT CCC TTG CTT GGC CTG GTC GGC GCC AGC GGT 220
Leu Leu Trp Leu Leu Leu Pro Leu Leu Gly Leu Val Gly Ala Ser Gly
            15                  20                  25

CCC CGT ACC TTA GTG CTT CTG GAC AAC CTC AAC CTG CGG GAG ACG CAT 268
Pro Arg Thr Leu Val Leu Leu Asp Asn Leu Asn Leu Arg Glu Thr His
        30                  35                  40

TCA CTT TTC TTC CGG AGC CTA AAG GAT CGG GGC TTC GTA CTC ACA TTC 316
Ser Leu Phe Phe Arg Ser Leu Lys Asp Arg Gly Phe Val Leu Thr Phe
    45                  50                  55

AAG ACC GCA GAT GAC CCC AGC CTG TCC CTG ATT AAG TAC GGA GAG TTC 364
Lys Thr Ala Asp Asp Pro Ser Leu Ser Leu Ile Lys Tyr Gly Glu Phe
60                  65                  70                  75

CTC TAT GAC AAT CTC ATC GTC TTT TCA CCT TCG GTA GAA GAT TTT GGA 412
Leu Tyr Asp Asn Leu Ile Val Phe Ser Pro Ser Val Glu Asp Phe Gly
                80                  85                  90

GGA AAT ATC AAC GTG GAG ACC ATC AGT ACC TTT ATC GAC GGC GGA GGC 460
Gly Asn Ile Asn Val Glu Thr Ile Ser Thr Phe Ile Asp Gly Gly Gly
            95                  100                 105

AGT GTC CTG GTA GCT GCC AGC TCA GAC ATC GGT GAC CCT CTC CGC GAG 508
Ser Val Leu Val Ala Ala Ser Ser Asp Ile Gly Asp Pro Leu Arg Glu
        110                 115                 120

CTG GGC AGT GAG TGT GGG ATT GAG TTT GAC GAG GAG AAA ACG GCC GTC 556
Leu Gly Ser Glu Cys Gly Ile Glu Phe Asp Glu Glu Lys Thr Ala Val
    125                 130                 135

ATT GAC CAT CAC AAC TAT GAT GTC TCA GAC CTC GGC CAG CAC ACG CTC 604
Ile Asp His His Asn Tyr Asp Val Ser Asp Leu Gly Gln His Thr Leu
140                 145                 150                 155

ATT GTG GCC GAC ACT GAG AAC CTG CTG AAG GCC CCG ACC ATC GTC GGG 652
Ile Val Ala Asp Thr Glu Asn Leu Leu Lys Ala Pro Thr Ile Val Gly
                160                 165                 170

AAG TCA TCT CTG AAT CCC ATC CTC TTC CGA GGT GTT GGG ATG GTG GCT 700
Lys Ser Ser Leu Asn Pro Ile Leu Phe Arg Gly Val Gly Met Val Ala
            175                 180                 185

GAT CCT GAC AAT CCT TTG GTG CTG GAC ATC CTG ACC GGC TCT TCT ACC 748
Asp Pro Asp Asn Pro Leu Val Leu Asp Ile Leu Thr Gly Ser Ser Thr
        190                 195                 200

TCT TAC TCC TTC TTC CCA GAT AAA CCC ATC ACG CAG TAC CCG CAC GCG 796
Ser Tyr Ser Phe Phe Pro Asp Lys Pro Ile Thr Gln Tyr Pro His Ala
    205                 210                 215

GTG GGG AAG AAC ACG CTG CTC ATC GCG GGG CTG CAG GCC CGG AAC AAC 844
Val Gly Lys Asn Thr Leu Leu Ile Ala Gly Leu Gln Ala Arg Asn Asn
220                 225                 230                 235

GCC CGT GTC ATC TTC AGC GGC TCC CTC GAC TTC TTC AGC GAT GCC TTC 892
Ala Arg Val Ile Phe Ser Gly Ser Leu Asp Phe Phe Ser Asp Ala Phe
                240                 245                 250

TTC AAC TCC GCG GTG CAG AAG GCC ACG CCT GGC TCC CAG AGG TAT CCC 940
Phe Asn Ser Ala Val Gln Lys Ala Thr Pro Gly Ser Gln Arg Tyr Pro
            255                 260                 265

CAG ACA GGC AAC TAT GAG CTC GCC GTG GCC CTC TCC CGC TGG GTG TTC 988
Gln Thr Gly Asn Tyr Glu Leu Ala Val Ala Leu Ser Arg Trp Val Phe
        270                 275                 280

AAG GAG GAG GGT GTC CTC CGA GTG GGG CCT GTG TCC CAC CAT CGG GTG 1036
Lys Glu Glu Gly Val Leu Arg Val Gly Pro Val Ser His His Arg Val
    285                 290                 295

GGC GAG AAA GCC CCA CCC AAC GCC TAC ACC GTC ACT GAC CTA GTC GAG 1084
Gly Glu Lys Ala Pro Pro Asn Ala Tyr Thr Val Thr Asp Leu Val Glu
300                 305                 310                 315

TAC AGC ATC GTG ATT GAG CAG CTC TCA CAG GGC AGA TGG GTC CCC TTT 1132
Tyr Ser Ile Val Ile Glu Gln Leu Ser Gln Gly Arg Trp Val Pro Phe
                320                 325                 330

GAT GGC GAC GAC ATT CAG CTG GAG TTT GTC CGC ATC GAT CCT TTC GTG 1180
Asp Gly Asp Asp Ile Gln Leu Glu Phe Val Arg Ile Asp Pro Phe Val
            335                 340                 345

AGG ACC TTC TTG AAG AGG AAA GGC GGC AAG TAC AGC GTC CAG TTC AAG 1228
Arg Thr Phe Leu Lys Arg Lys Gly Gly Lys Tyr Ser Val Gln Phe Lys
        350                 355                 360

TTG CCG GAC GTG TAC GGC GTG TTC CAG TTC AAA GTG GAC TAC AAC CGG 1276
Leu Pro Asp Val Tyr Gly Val Phe Gln Phe Lys Val Asp Tyr Asn Arg
    365                 370                 375

CTG GGC TAC ACG CAC CTG TAC TCC TCC ACT CAG GTG TCC GTG CGG CCC 1324
Leu Gly Tyr Thr His Leu Tyr Ser Ser Thr Gln Val Ser Val Arg Pro
380                 385                 390                 395

CTG CAG GCA CAC GCA GTA CGA GCG CTT CAT CCC CTC GGC TTA CCC CTA 1372
Leu Gln Ala His Ala Val Arg Ala Leu His Pro Leu Gly Leu Pro Leu
                400                 405                 410

CTA CGC CAG CGC CTT CTC CAT GAT GGT CGG GCT CTT CAT CTT CAG CGT 1420
Leu Arg Gln Arg Leu Leu His Asp Gly Arg Ala Leu His Leu Gln Arg
            415                 420                 425

CGT CTT CTT GCA CAT GAA GGA GAA GGA GAA GTC TGACTGAGGG GCCGGGCCGG 1473
Arg Leu Leu Ala His Glu Gly Glu Gly Glu Val
        430                 435

GCCCCAGGAC TCCTTACAAC ACACAGGGAG GGTTTTTATA GGCTTGCCTT CCCCCCCCTT 1533

TATGGTGGGC TTTGTTTGTT TTTAAAGCCA CGGACAATGG CACAGCTTAC CTCAGTGGGA 1593

GATGCAAGAT GAGTACCAGG GGGTGGTTAG GAATAATTTC TAAGTTTTTC CACCTTGAAT 1653

GCTGAGTGGT ATTTTTCATA TGTAAAGTCA ACTGATTTCT AAAATAAAAG AAAAACATCA 1713

CCCTCAGAAA AAAAAAA 1730

(2) INFORMATION FOR SEQ ID NO:2:

(i) SEQUENCE CHARACTERISTICS:
(A) LENGTH: 438 amino acids
(B) TYPE: amino acid
(D) TOPOLOGY: linear

(ii) MOLECULE TYPE: protein

(xi) SEQUENCE DESCRIPTION: SEQ ID NO:2:

Met Glu Leu Gly Ala Ala Ala Arg Ala Trp Ser Leu Leu Trp Leu Leu
1               5                   10                  15

Leu Pro Leu Leu Gly Leu Val Gly Ala Ser Gly Pro Arg Thr Leu Val
            20                  25                  30

Leu Leu Asp Asn Leu Asn Leu Arg Glu Thr His Ser Leu Phe Phe Arg
        35                  40                  45

Ser Leu Lys Asp Arg Gly Phe Val Leu Thr Phe Lys Thr Ala Asp Asp
    50                  55                  60

Pro Ser Leu Ser Leu Ile Lys Tyr Gly Glu Phe Leu Tyr Asp Asn Leu
65                  70                  75                  80

Ile Val Phe Ser Pro Ser Val Glu Asp Phe Gly Gly Asn Ile Asn Val
                85                  90                  95

Glu Thr Ile Ser Thr Phe Ile Asp Gly Gly Gly Ser Val Leu Val Ala
            100                 105                 110

Ala Ser Ser Asp Ile Gly Asp Pro Leu Arg Glu Leu Gly Ser Glu Cys
        115                 120                 125

Gly Ile Glu Phe Asp Glu Glu Lys Thr Ala Val Ile Asp His His Asn
    130                 135                 140

Tyr Asp Val Ser Asp Leu Gly Gln His Thr Leu Ile Val Ala Asp Thr
145                 150                 155                 160

Glu Asn Leu Leu Lys Ala Pro Thr Ile Val Gly Lys Ser Ser Leu Asn
                165                 170                 175

Pro Ile Leu Phe Arg Gly Val Gly Met Val Ala Asp Pro Asp Asn Pro
            180                 185                 190

Leu Val Leu Asp Ile Leu Thr Gly Ser Ser Thr Ser Tyr Ser Phe Phe
        195                 200                 205

Pro Asp Lys Pro Ile Thr Gln Tyr Pro His Ala Val Gly Lys Asn Thr
    210                 215                 220

Leu Leu Ile Ala Gly Leu Gln Ala Arg Asn Asn Ala Arg Val Ile Phe
225                 230                 235                 240

Ser Gly Ser Leu Asp Phe Phe Ser Asp Ala Phe Phe Asn Ser Ala Val
                245                 250                 255

Gln Lys Ala Thr Pro Gly Ser Gln Arg Tyr Pro Gln Thr Gly Asn Tyr
            260                 265                 270

Glu Leu Ala Val Ala Leu Ser Arg Trp Val Phe Lys Glu Glu Gly Val
        275                 280                 285

Leu Arg Val Gly Pro Val Ser His His Arg Val Gly Glu Lys Ala Pro
    290                 295                 300

Pro Asn Ala Tyr Thr Val Thr Asp Leu Val Glu Tyr Ser Ile Val Ile
305                 310                 315                 320

Glu Gln Leu Ser Gln Gly Arg Trp Val Pro Phe Asp Gly Asp Asp Ile
                325                 330                 335

Gln Leu Glu Phe Val Arg Ile Asp Pro Phe Val Arg Thr Phe Leu Lys
            340                 345                 350

Arg Lys Gly Gly Lys Tyr Ser Val Gln Phe Lys Leu Pro Asp Val Tyr
        355                 360                 365

Gly Val Phe Gln Phe Lys Val Asp Tyr Asn Arg Leu Gly Tyr Thr His
    370                 375                 380

Leu Tyr Ser Ser Thr Gln Val Ser Val Arg Pro Leu Gln Ala His Ala
385                 390                 395                 400

Val Arg Ala Leu His Pro Leu Gly Leu Pro Leu Leu Arg Gln Arg Leu
                405                 410                 415
Leu His Asp Gly Arg Ala Leu His Leu Gln Arg Arg Leu Leu Ala His
            420                 425                 430
Glu Gly Glu Gly Glu Val
        435